# EUROPÄISCHE PATENTSCHRIFT

(11) **EP 3 160 630 B1**
(45) Veröffentlichungstag und Bekanntmachung des Hinweises auf die Patenterteilung: **31.08.2022**
(21) Anmeldenummer: 15730179.7
(22) Anmeldetag: 22.06.2015
(51) Int. Cl.: B01J 8/00, B01J 19/00, C07C 201/08

(54) **VERFAHREN ZUR HERSTELLUNG VON CHEMISCHEN PRODUKTEN MIT BETRIEBSUNTERBRECHUNGEN**
PROCESS FOR MANUFACTURING CHEMICAL PRODUCTS WITH STAND BY OF THE PROCESS
PROCÉDÉ DE FABRICATION DE PRODUITS CHIMIQUES AVEC DES INTERRUPTIONS DU PROCÉDÉ

(30) Priorität: 24.06.2014 EP 14173585
(43) Veröffentlichungstag der Anmeldung: 03.05.2017
(73) Patentinhaber: Covestro Intellectual Property GmbH & Co. KG, 51373 Leverkusen (DE)
(72) Erfinder: KNAUF, Thomas, 41542 Dormagen (DE); DRINDA, Peter, 47829 Krefeld (DE); WERSHOFEN, Stefan, 41065 Mönchengladbach (DE); GRUNER, Klaus-Gerd, 47239 Duisburg (DE); HARTJES, Volker, 47239 Duisburg (DE)
(74) Vertreter: Levpat
(86) Internationale Anmeldenummer: PCT/EP2015/063926
(87) Internationale Veröffentlichungsnummer: WO 2015/197522

(56) Entgegenhaltungen:
- WO-A1-2006/125759
- WO-A1-2013/053371
- WO-A1-2014/016292
- US-A1- 2005 227 129
- US-A1- 2008 262 112

## Beschreibung

Die vorliegende Erfindung betrifft Verfahren zur Herstellung von chemischen Produkten, bei denen der oder die Einsatzstoffe zu einem chemischen Produkt oder einer chemischen Zusammensetzung umgesetzt werden, wobei während einer Unterbrechung der Verfahren kein Eintrag von mindestens einem Einsatzstoff in die Reaktion stattfindet und die nicht von einer Revisions-, Wartungs-, Reparatur- oder Reinigungsmaßnahme betroffenen Anlagenteile in sogenannter Kreislauffahrweise betrieben werden. Dadurch wird unter anderem erreicht, dass nur das betroffene Anlagenteil für die Zeit der Maßnahme stillgelegt zu werden braucht, was vorteilhaft hinsichtlich Produktivität und Wirtschaftlichkeit des Verfahrens sowie der Qualität der hergestellten Produkte sein kann. Schließlich sind Verfahren zum Betrieb von Anlagen im Fall der Außerbetriebnahme einzelner Anlagenteile ein Gegenstand der Erfindung.

Großtechnische chemische Verfahren stehen zur Erzeugung von chemischen Zwischenprodukten und Endprodukten zur Verfügung. Die Verfahren können diskontinuierlich, semi-kontinuierlich, kontinuierlich oder in Kombination einer der drei Varianten betrieben werden. Die Verfahren sind endotherm oder exotherm und können isotherm oder adiabatisch durchgeführt werden. Die Durchführung des Verfahrens kann je nach chemischem Produkt in Gasphase, Flüssigphase mit und ohne Lösungsmittel oder in Schmelze erfolgen. Die Aufarbeitung und Reinigung des so erhaltenen chemischen Produktes kann nach einem der gängigen Verfahren der Technik wie z.B. Kristallisation, Wäsche oder Destillation oder in einer Kombination dieser Aufarbeitungsweisen erfolgen.

Chemische Zwischenprodukte und Endprodukte sind beispielsweise (Poly)Isocyanate und deren Vorprodukte, Polycarbonate und deren Vorprodukte, pharmazeutische Wirkstoffe und deren Vorprodukte oder Wirkstoffe für den Pflanzenschutz und deren Vorprodukte.

Die Güte eines Verfahrens zur Herstellung von chemischen Produkten ist einerseits definiert durch den Gehalt an unerwünschten Nebenprodukten im Produkt des Verfahrens. Andererseits ist die Güte eines Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt.

Idealerweise sind daher die großtechnischen Anlagen zur Durchführung solcher Herstellungsverfahren derart ausgelegt, dass bei einer passenden Qualität der eingesetzten Hilfs- und Einsatzstoffe und richtiger Wahl der Verfahrensparameter wie Druck, Temperatur, Mengenverhältnisse und Konzentrationen der Hilfs- und Einsatzstoffe etc. die Verfahren robust verlaufen. Das bedeutet, dass es in solchen kontinuierlich betriebenen Großanlagen idealerweise nicht zu Problemen wie etwa der Entstehung von Niederschlägen kommt, die sich am Anlagenequipment absetzen können oder Rohrleitungen verstopfen können.

Das An- und Abfahren von kontinuierlich betriebenen, chemischen Großanlagen stellt den Fachmann vor besondere Herausforderungen, bedingt durch die Probleme, die dabei spontan auftreten können. Solche Probleme umfassen beispielsweise ein "Einschlafen" der Reaktion, ein erhöhter Energiebedarf zum Starten der Reaktion, eine unvollständige Reaktion, die zur Überlastung der Aufarbeitung führen kann, die Entstehung von erhöhten Mengen an unerwünschten Nebenprodukten, die zu Qualitäts- und/oder Sicherheitsproblemen führen können, eine Desaktivierung, Schädigung oder Verkokung von Katalysatoren, eine Entstehung von Niederschlägen, die das Equipment verstopfen können, oder Anbackungen von Produkt, Nebenprodukten und/oder Substraten.

Produktionsstillstände, die Ab- und Anfahren der Anlage erforderlich machen, treten im industriellen Alltag immer wieder auf. Dabei kann es sich um einen Revisionsstillstand halten, der im Voraus geplant wird, wobei zu diesem Zweck die Anlage abgefahren, die Energien abgestellt und üblicherweise alle zu revidierenden Anlagenteile geöffnet und zum Zwecke der Prüfung gereinigt werden. Eine solche Revision kann eine oder mehrere Wochen dauern. Nach Beendigung der Revision wird die Produktionsanlage geschlossen, gegebenenfalls inertisiert, mit Hilfsstoffen versehen und nachdem die entsprechenden Energien und Rohstoffe zur Verfügung stehen, kann die Produktionsanlage wieder angefahren werden. Ein Produktionsstillstand ist allerdings nicht zwangsläufig mit einem Öffnen oder einem anderen mechanischen Eingriff in einen Reaktor oder einen anderen Apparat der Anlage verbunden, sondern kann auch mit dem Abstellen und erneuten Starten der Produktionsanlage aus diversen anderen Gründen in Verbindung stehen, wie z. B. bei einem Ausfall der Rohstoffversorgung. In einem solchen Fall wird die Anlage üblicherweise im Teillastbetrieb gefahren und muss im schlimmsten Fall, wenn die logistische Versorgungskette unterbrochen ist, abgestellt werden. Des Weiteren können Produktionsstillstände durch Wartungs-, Reinigungs- oder Reparaturbedarf an der Produktionsanlage erzwungen sein. Man spricht beispielsweise im Nitrobenzolprozess von Kurzstillständen, wenn die Produktion bis zu einem Tag unterbrochen ist. Alle diese Produktionsstillstände zeichnen sich in der Praxis dadurch aus, dass es zu Produktionsverlusten kommt, und dass beim Wiederanfahren der Anlage, wenn beispielsweise inertisiert werden muss, Stickstoff verbraucht wird oder beim Aufheizen der Anlage oder der Einsatzstoffe Energie in Form von Dampf und Strom zugeführt werden muss.

Dem Fachmann ist bekannt, dass ein scmikontinuicrlich oder kontinuierlich betriebener industrieller Prozess ausgehend von einer in Betrieb befindlichen Produktionsanlage nicht augenblicklich in einen Produktionsstillstand überführt werden kann, sondern vorher kontrolliert abgefahren werden muss. Dies gilt auch für ein Anlagenaus im Falle einer Havarie. Um nach dem Produktionsstillstand wieder produzieren zu können, muss die Anlage wieder auf die Prozessparameter vor dem Produktionsstillstand hochgefahren werden. Edukte und Apparaturen müssen aufgeheizt werden, Apparate müssen gegebenenfalls inertisiert werden, die Belastung der Apparate mit den Edukten wird allmählich auf den angestrebten Soll-Wert gesteigert. Während dieser Anfahrphase geht also weiterhin Produktion verloren, und es muss überproportional viel Energie aufgewendet werden, um die ausgekühlte Anlage zum Anfahren vorzubereiten und sie dann auch unter Beachtung aller betriebsrelevanten Parameter auf den gewünschten Sollwert hochzufahren.

Nachteilig ist des Weiteren, dass bei notwendigen Wartungs-, Reparatur- und Reinigungsarbeiten an oder in einem Reaktor oder einem sonstigen Anlagenteil regelmäßig immer alle Anlagenteile abgeschaltet werden müssen, da die Verfahrensschritte aufeinander aufbauen und somit immer sukzessive erfolgen. Dadurch muss die gesamte Anlage geleert werden, was zu einem erheblichen Materialausschuss führt. Des Weiteren muss Energie aufgewendet werden, um Reaktoren und Anlagenteile wieder auf die jeweiligen Betriebstemperaturen zu bringen. Solche Produktionsstillstände für Anlagenrevisionen, Reparatur-, Wartungs- und Reinigungsmaßnahmen oder auftretende Rohstoff- oder Hilfsstoffmängel, geplant oder ungeplant, sind daher immer wiederkehrende Anlagenzustände, die einen erheblichen Einfluss auf das wirtschaftliche Betreiben einer kontinuierlich arbeitenden Anlage bzw. eines kontinuierlich arbeitenden Verfahrens haben.

Den heutigen Verfahren des Standes der Technik zur großtechnischen Herstellung chemischer Produkte gelingt es zwar im Allgemeinen, mit hoher Ausbeute die gewünschten Produkte herzustellen, ohne dass es bei den jeweiligen Endprodukten zu einer signifikanten Qualitätseinbuße kommt, jedoch werden in der Literatur fast ausschließlich Verfahren beschrieben, die sich im Normalbetrieb befinden.

Es liegen nur für einige wenige großchemische Prozesse Beschreibungen zur Vermeidung von Problemen während der An- und Abfahrphase dieser Prozesse vor. So beschreibt WO 2014/016292 A1 das Anfahren des Prozesses zur Nitrobenzolherstellung, wobei als Einsatzstoff ein Benzol, das weniger als 1,5% Aliphaten enthält, eingesetzt wird, wodurch unter anderem für eine verbesserte Reaktionsgeschwindigkeit der Reaktion Sorge getragen wird, wodurch Dampf eingespart wird und die Entstehung des Nebenproduktes Pikrinsäure minimiert wird. Ähnliche positive Effekte beschreibt die WO 2014/016290 A1 zum Anfahrprocedere der Nitrierung von Benzol zu Nitrobenzol, wobei eine Kreislaufschwefelsäure, die weniger als 1.0% Nitrobenzol enthält, verwendet wird, wodurch unter anderem Dampf eingespart wird, da die Aufarbeitung nicht durch zusätzliches Benzol, das durch unvollständige Nitrierung anfällt, belastet wird und die Entstehung der Nebenprodukte Dinitrobenzol und Pikrinsäure minimiert wird.

Die großtechnische kontinuierliche Herstellung von Anilin durch Hydrierung von Nitrobenzol ist in der WO 2013/156410 A1 beschrieben, wobei speziell Bezug genommen wird auf das Anfahren des Hydrierprozesses nach einer Regeneration des Hydrierkatalysators. Dabei wird ein Wasseraustausch im Flüssigringverdichter vorgenommen, um darin vorhandenes Kohlendioxid separat zu entsorgen, wodurch Verstopfungen und Korrosion im Abluftsystem durch Ammoniumcarbonat vermieden werden. WO 2013/156409 A1 beschreibt ebenfalls die Herstellung von Anilin, wobei das Augenmerk auf das Abfahren der Hydrierung von Nitrobenzol gelegt wird und das erfinderische Vorgehen beim Abfahren der Reaktion beinhaltet, dass Wasserstoff lange genug in genügender Menge in den Reaktionsräumen vorhanden ist bis sämtliche Nitroaromaten mit Wasserstoff abreagiert haben und somit während des Abfahrvorganges der Hydrierung komplett aufgebraucht werden. Dadurch werden eine verstärkte Nebenproduktbildung und eine Verkokung des Katalysators durch Nitrobenzolreste, die mit zu geringem Wasserstoffüberschuss hydriert werden, verhindert. Darüber hinaus wird der Aufwand für die Katalysatorregenerierung minimiert und die Laufzeit der Reaktoren verlängert.

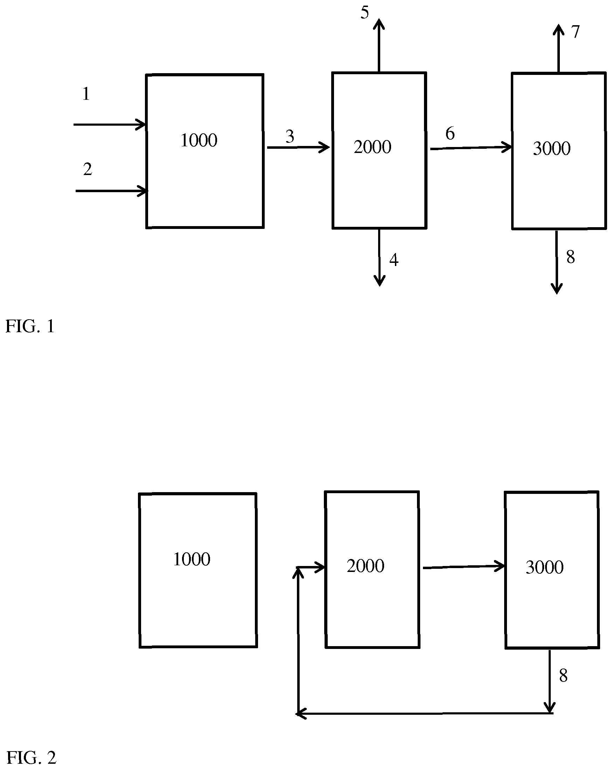

Die Patentanmeldung WO 2006/125759 A1 befasst sich mit einer Fischer-Tropsch-Produktionsanlage sowie einem Verfahren zur Herstellung von Kohlenwasserstoffen. Die Patentanmeldung befasst sich unter anderem damit, wie im Falle gestörter Methanverfügbarkeit der Produktionsbetrieb für begrenzte Zeit wenigstens eingeschränkt weiter aufrechterhalten werden kann, bzw. wie die Produktionsanlage kontrolliert heruntergefahren werden kann. Dies wird u. a. erreicht durch Bereitstellung eines Vorratstanks 20 für verflüssigtes Erdgas. Es wird erwähnt, dass es vorteilhaft sein kann, beispielsweise bei Notabschaltung der Vergasungseinrichtung, diese auf relativ hoher Temperatur zu halten, um die spätere Wiederinbetriebnahme schneller bewerkstelligen zu können (sog. "*hot standby*"). Es wird ferner eine Ausführungsform beschrieben, in der eine Destillationseinrichtung 18 einem Hydrocracking-Reaktor 16 nachgeschaltet ist und ein *Teils*trom des Austrags der Destillationseinrichtung 18 in den Hydrocracking-Reaktor zurückgeführt werden kann, während der übrige Austrag der Destillationseinrichtung 18 seinem normalen Verwendungszweck zugeführt wird (vgl. FIG. 1). Eine allgemeine Lehre zum Kreislaufbetrieb von *einzelnen* Anlagenteilen während der *zeitgleichen vollständigen* Außerbetriebnahme von *anderen* Anlagenteilen so, dass in diesen anderen Anlagenteilen bspw. Wartungsarbeiten durchgeführt werden können, ist dieser Patentanmeldung nicht zu entnehmen.

Die Patentanmeldung US 2008/026112 A1 befasst sich ebenfalls mit Fischer-Tropsch-Verfahren, und zwar speziell mit dem Fall der zeitweiligen Produktionsunterbrechung in einer dreiphasigen Reaktionszone umfassend eine Flüssigphase, eine Gasphase und eine Phase mit suspendierten Katalysatorpartikeln. Es wird offenbart, die Eduktzufuhr zu unterbrechen und die Reaktionszone mit einem Inhibitionsgas zu inertisieren. Es wird eine Ausführungsform beschrieben, in welcher das Inhibitionsgas in den unteren Teil der Reaktionszone zurückgeführt wird, um die Katalysatorpartikel in Suspension zu halten. Im Allgemeinen folgen dabei nach der Reaktion ein Kondensationsschritt und eine Trennschritt, wobei dann die Gasphase aus dem Trennschritt in den unteren Teil des Reaktors zurückgeführt wird. Zum Wiederanfahren der Reaktion wird die Reaktionszone mit einem Aktivierungsgas beschickt. Eine allgemeine Lehre zum Kreislaufbetrieb von *einzelnen* Anlagenteilen während der *zeitgleichen vollständigen* Außerbetriebnahme von *anderen* Anlagenteilen so, dass in diesen anderen Anlagenteilen bspw. Wartungsarbeiten durchgeführt werden können, ist dieser Patentanmeldung nicht zu entnehmen.

Die Patentanmeldung WO 2013/053371 A1 befasst sich mit einem Verfahren zum Bereitstellen eines methanreichen Produktgases sowie einer dazu geeigneten Anordnung. Bei Wasserstoffmangel wird der Reaktor im Standby (ohne weitere kontinuierliche Eduktgaszufuhr) betrieben. Beim Wechsel zwischen Normalbetrieb des Reaktors und Bereitschaftszustand wird zeitweise ein Produktgas gebildet, das den üblichen Qualitätsanforderungen nicht genügt (auch als "Schlechtgas" bezeichnet). Dieses Schlechtgas wird *nach Wiederaufnahme des Normalbetriebs* wenigstens teilweise zu einem vor der Methanisierung erfolgenden Verfahrensschritt zurückgeführt. Dies geschieht bevorzugt durch zeitversetzte graduelle Einspeisung zwischengelagerten Schlechtgases. Zum Standby-Betrieb selbst macht die Patentanmeldung kaum Angaben. Es wird lediglich offenbart, dass der Reaktor gespült werden kann, bspw. mit Wasserstoffgas. Eine allgemeine Lehre zum Kreislaufbetrieb von *einzelnen* Anlagenteilen während der *zeitgleichen vollständigen* Außerbetriebnahme von *anderen* Anlagenteilen so, dass in diesen anderen Anlagenteilen bspw. Wartungsarbeiten durchgeführt werden können, ist dieser Patentanmeldung nicht zu entnehmen.

Die Patentanmeldung US 2005/0227129 A1 befasst sich mit einer Aufheizvorrichtung für Brennstoffzellenkraftwerke. Das Brennstoffzellenkraftwerk umfasst - vgl. FIG. 1 und die zugehörigen Textpassagen - drei Reaktoren, nämlich einen Reformer (**3**), einen Kohlenstoffmonoxid-Konverter (**4**) zur Umsetzung von Kohlenstoffmonoxid mit Wasserdampf und einen weiteren Konverter (**5**) zur Umsetzung von Kohlenstoffmonoxid mit Sauerstoff. In allen diesen Reaktoren finden chemische Reaktionen statt. Sie sind ohne bauliche Umbaumaßnahmen nicht dazu geeignet, das Verfahrensprodukt einer chemischen Umsetzung *unter Abtrennung von Nebenströmen* aufzuarbeiten und zu reinigen. Die Aufheizvorrichtung gemäß US 2005/0227129 A1 umfasst einen Brenner (**6**) und Leitungen (**71** bis **73**) für die Zufuhr heißer Verbrennungsgase zwecks Aufheizung des Brennstoffzellenkraftwerks. Eine *gezielte* Rückführung des Ausgangsstroms eines Apparats durch diese Leitungen als Eingangsstrom in diesen oder einen vorgeschalteten Apparat ist offensichtlich nicht vorgesehen und erscheint auch ohne weitere Umbaumaßnahmen wie den Einbau zusätzlicher Ventile nicht möglich. Somit ist auch dieser Patentanmeldung keine allgemeine Lehre zum Kreislaufbetrieb von *einzelnen* Anlagenteilen während der *zeitgleichen vollständigen* Außerbetriebnahme von *anderen* Anlagenteilen so, dass in diesen anderen Anlagenteilen bspw. Wartungsarbeiten durchgeführt werden können, zu entnehmen.

Wünschenswert wären also Verfahren und Anlagen zur Herstellung von chemischen Produkten, bei denen es möglich ist, Produktionsstillstände beim Betreiben der jeweiligen Prozesse hinsichtlich des Zeitaufwandes und gegebenenfalls auch hinsichtlich des Energie- und Materialverbrauches zu optimieren. Diese würden in einem nicht unerheblichen Umfang zu einer Verbesserung der Produktivität und somit der Wirtschaftlichkeit von großtechnischen chemischen Herstellungsprozessen und den entsprechenden Anlagen dafür führen.

Überraschend wurde gefunden, dass diese Aufgabe gelöst werden kann, wenn (vereinfacht ausgedrückt und ohne hierauf beschränkt zu sein) während eines Kurzstillstandes so viele Anlagenteile wie möglich in "Kreislauffahrweise" ("stand-by") gestellt werden, um nach der Maßnahme die Gesamtanlage unverzüglich wieder anfahren zu können. Überraschend wurde auch gefunden, dass der Energieverbrauch bei in Kreislauf gestellter Anlage unter Umständen geringer ist als bei Komplett-Abschaltung und Wiederinbetriebnahme der Gesamt-Anlage. Durch eine gezielte Kreislauffahrweise der nicht von dem Kurzstillstand betroffenen Anlagenteile werden vielfältige Vorteile realisiert, wie weiter unten noch näher erläutert wird.

Die vorliegende Erfindung stellt daher Folgendes bereit:
Ein **Verfahren zur Herstellung eines chemischen Produktes oder einer chemischen Zusammensetzung,** wobei das Verfahren die nachfolgenden Schritte umfasst und in einer Anlage aufweisend diesen Schritten entsprechende Anlagenteile durchgeführt wird:
(I) Reaktion von mindestens einem Substrat in einem Reaktor unter Bildung mindestens eines chemischen Produktes oder einer chemischen Zusammensetzung, wobei das mindestens eine Substrat mit einem Massenstrom m₁ in den Reaktor eingetragen wird;
(II) Aufarbeitung des in Schritt (I) erhaltenen Reaktionsgemisches in einem Aufarbeitungsapparat unter Erhalt eines Rohprodukts und mindestens eines Nebenstroms umfassend die vom Rohprodukt abgetrennten Bestandteile;
(III) Reinigung des in Schritt (II) erhaltenen Rohproduktes in mindestens einem Reinigungsapparat unter Erhalt eines gereinigten Endprodukts und mindestens eines Nebenstroms umfassend die vom gereinigten Endprodukt abgetrennten Bestandteile; und die optionalen Schritte (IV) bis (V)
(IV) Aufarbeitung des in Schritt (II) erhaltenen mindestens einen Nebenstroms in einer Aufarbeitungseinrichtung;
(V) Aufarbeitung des in Schritt (III) erhaltenen mindestens einen Nebenstroms in einer Aufarbeitungseinrichtung,
wobei
bei einem Produktionsstillstand einer Dauer von 1 Stunde bis 5 Tagen zu Revisions-, Reparatur-, Reinigungs- oder Wartungszwecken in Teilen der Anlage eines oder mehrere, jedoch nicht alle, Anlagenteile aus den Schritten (I) bis (V), insofern diese durchgeführt werden, außer Betrieb genommen werden und in diesen Anlagenteilen eine Revisions-, Reparatur-, Reinigungs- oder Wartungsmaßnahme durchgeführt wird, und wobei der Massenstrom m₁ in Schritt (I) auf null reduziert und in jedem der nicht außer Betrieb genommenen Anlagenteile der Ausgangsstrom des Anlagenteils wieder als Eingangsstrom des jeweiligen Anlagenteiles oder eines davor liegenden Anlagenteils verwendet wird, wobei nach der Maßnahme die gesamte Anlage unverzüglich wieder angefahren wird. Dabei umfasst das erfindungsgemäße Verfahren bevorzugt die Schritte (I) bis (IV) und besonders bevorzugt die Schritte (I) bis (V).

Ein weiterer Gegenstand der vorliegenden Erfindung ist ein **Verfahren zum Betrieb einer Anlage zur Herstellung eines chemischen Produkts oder einer chemischen Zusammensetzung,** was weiter unten noch im Detail beschrieben wird.

Das mindestens eine *"Substrat"* bezeichnet einen beliebigen chemischen Ausgangstoff. So ist es bspw. denkbar, ein (d. h. nur ein) Substrat 1 ohne weitere Reaktanden durch Einwirkung erhöhter Temperatur und/oder erhöhten Drucks zum gewünschten Produkt umzusetzen. In einer anderen, bevorzugten Ausführungsform können im Reaktor aus Schritt (I) auch mehrere Substrate miteinander umgesetzt werden. In einer Ausführungsform der vorliegenden Erfindung können beispielsweise mindestens zwei Substrate im Reaktor aus Schritt (I) zur Reaktion gebracht werden, wobei das zweite Substrat 2 mit einem Massenstrom m₂ in den Reaktor aus Schritt (I) eingebracht wird. Bevorzugt werden dann bei Außerbetriebnahme eines Anlagenteils beide Massenströme, m₁ und m₂, auf null reduziert. Reagieren *n* Substrate *i* in Schritt (I), wobei *n* eine natürliche Zahl von bevorzugt 1 bis 5, besonders bevorzugt 1 bis 4, ganz besonders bevorzugt 1 bis 3, ist, so werden bei der Außerbetriebnahme eines Anlagenteils bevorzugt alle *n* Massenströme m*ᵢ* auf null reduziert.

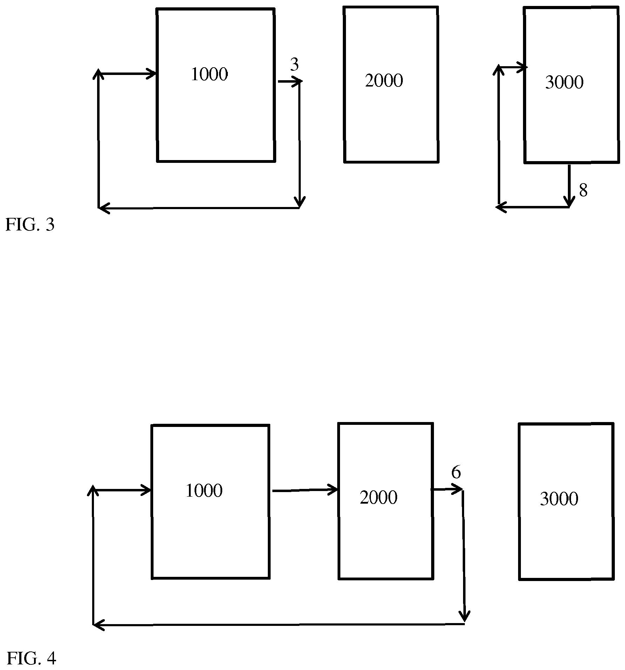

Unter einer *"Außerbetriebnahme"* eines Anlagenteils wird dabei dessen Stilllegung so verstanden, dass in dem Anlagenteil eine Revisions-, Reparatur-, Wartungs- oder Reinigungsmaßnahme durchgeführt werden kann. Die vorliegende Erfindung ermöglicht es, dass bei einer Revisions-, Reparatur-, Wartungs- oder Reinigungsmaßnahme in einem Anlagenteil nicht die gesamte Produktionsanlage außer Betrieb genommen werden muss. Vielmehr ermöglicht es die vorliegende Erfindung, dass nicht von der Revisions-, Reparatur-, Wartungs- oder Reinigungsmaßnahme betroffene Anlagenteile bzw. die entsprechenden Verfahrensschritte in eine "Kreislauffahrweise" betrieben werden können. Da bei Außerbetriebnahme eines Anlagenteils erfindungsgemäß der Massenstrom m₁ auf null reduziert wird, steht die Produktion währenddessen still. Darunter ist ein verhältnismäßig kurzer (mit einer Dauer von 1 Stunde bis 5 Tagen, bevorzugt von 1 Stunde bis 3 Tagen und besonders bevorzugt von 1 Stunde bis 2 Tagen) Produktionsstillstand zu Revisions-, Reparatur-, Reinigungs- oder Wartungszwecken in Teilen der Anlage zu verstehen. Die vorliegende Erfindung befasst sich nicht mit dem Fall, dass, etwa für eine Anlagenrevision, eine Produktionsanlage *vollständig* (d. h. *alle* Anlagenteile) außer Betrieb genommen werden muss. Der Begriff *"Außerbetriebnahme"* umfasst demnach erfindungsgemäß bei Vorhandensein von *m Anlagenteilen im Sinne der vorliegenden Erfindung* (siehe hierzu auch den folgenden Absatz), wobei *m* eine natürliche Zahl ist, die Außerbetriebnahme von maximal *m* - 1 dieser Anlagenteile. Erfindungsgemäß wird also mindestens ein Anlagenteil **nicht** "*außer Betrieb genommen* " (d. h. *"vollständig stillgelegt"*). Bevorzugt befasst sich die vorliegende Erfindung mit dem Fall der Außerbetriebnahme von 1 bis 2 Anlagenteilen, besonders bevorzugt von 1 Anlagenteil. Erfindungsgemäß wird daher bei Außerbetriebnahme eines Anlagenteils (oder mehrerer Anlagenteile, jedoch nicht aller Anlagenteile) in jedem Fall die Bildung weiteren Produkts unterbrochen (da der Massenstrom des mindestens einen Substrats m₁ auf null reduziert wird und daher kein weiteres Produkt mehr produziert werden kann). Erfindungsgemäß umfasst ist aber auch der Fall, dass der Reaktor aus Schritt (I) in *Kreislauffahrweise* (siehe hierzu auch den folgenden Absatz) betrieben und *ein anderes Anlagenteil* im Sinne der zuvor genannten Definition *außer Betrieb genommen* wird (vgl. z. B. FIG. 3).

Unter "Kreislauffahrweise" wird im Rahmen dieser Erfindung verstanden, dass der Ausgangstrom eines Anlagenteils als Eingangsstroms dieses Anlagenteils oder eines davorliegenden (d. h. strömungstechnisch davorliegenden) Anlagenteils verwendet wird. Dabei ist mit *"Anlagenteil"* das dem jeweiligen Schritt (I) bis (V), insofern diese durchgeführt werden, entsprechende Anlagenteil einer Anlage zur Herstellung von chemischen Produkten nach dem erfindungsgemäßen Verfahren gemeint. So umfasst beispielsweise das Anlagenteil des Schritts (I) *"einen Reaktor"*, wobei dieser Begriff auch solche Ausführungsformen umfasst, in denen mehrere Reaktoren (beispielsweise eine Kaskade mehrere in Serie geschalteter Reaktoren) eingesetzt werden (mit anderen Worten, das Wort "ein" ist diesem Zusammenhang und im Zusammenhang mit anderen Apparaten auch anderer Anlagenteile als unbestimmter Artikel und nicht als Zahlwort aufzufassen). Parallel geschaltete oder in Reihe geschaltete Reaktoren sind im Stand der Technik auch in der Herstellung von chemischen Produkten bekannt und können in bestimmten Dimensionierungen und betrieblichen Eigenarten auch Vorteile mit sich bringen. Die erfindungsgemäße Anlage und das erfindungsgemäße Verfahren sehen daher auch bevorzugte Ausführungsformen vor, in denen in Reihe oder parallel geschaltete Reaktoren, insbesondere im Schritt (I) bevorzugt angewendet werden.

Ein Anlagenteil aus einem der Schritte (I) bis (V) kann also mehrere, auch unterschiedliche, Apparate umfassen.

Das Anlagenteil aus Schritt (II) umfasst einen Aufarbeitungsapparat, z. B. einen Phasentrennapparat zur Auftrennung eines mehrphasigen Rohprodukts in bspw. eine wässrige, Reaktionswasser enthaltende Phase, eine organische, das gewünschte Produkt enthaltende Phase und eine Gasphase enthaltend gasförmige Neben- und/oder Koppelprodukte sowie ggf. zugesetzte Inertgase.

Solche Nebenströme wie ein wässriger Strom aus Reaktionswasser und ein gasförmiger Strom aus Neben- und/oder Koppelprodukten sowie ggf. zugesetzten Inertgases werden bevorzugt in Schritt (IV) weiter aufgearbeitet. Das Anlagenteil aus Schritt (IV) umfasst zu diesem Zweck eine Aufarbeitungseinrichtung. Diese Aufarbeitungseinrichtung kann verschiedene einzelne Apparate umfassen, wie bspw. eine Vorrichtung zur Destillation oder Strippung des mindestens einen Nebenstroms aus Schritt (II). Schritt (IV) kann auch mehrfach vorkommen. Als Beispiel sei der Fall genannt, dass die Aufarbeitung von Nebenströmen aus Schritt (II) auf mehrere Anlagenteile verteilt ist. Dies kann insbesondere dann der Fall sein, wenn ein Anlagenteil für die Aufarbeitung eines flüssigen Nebenstroms und ein Anlagenteil für die Aufarbeitung eines gasförmigen Nebenstroms vorgesehen ist. In diesem beispielhaft genannten Fall umfasst das Verfahren zwei Schritte (und Anlagenteile) der Kategorie (IV), welche parallel nebeneinander durchgeführt werden, nämlich die Aufarbeitung des flüssigen Nebenstroms in einer Aufarbeitungseinrichtung (Schritt (IVa)) und die Aufarbeitung des gasförmigen Nebenstroms in einer weitern Aufarbeitungseinrichtung (Schritt (IVb)).

Das Anlagenteil aus Schritt (III) umfasst einen Rcinigungsapparat, z. B. eine Destillationsapparatur.

Der bei der Reinigung in Schritt (III) abgetrennte mindestens eine Nebenstrom wird bevorzugt in Schritt (V) weiter aufgearbeitet. Das Anlagenteil aus Schritt (V) umfasst zu diesem Zweck eine Aufarbeitungseinrichtung. Diese Aufarbeitungseinrichtung kann verschiedene einzelne Apparate umfassen, wie bspw. eine Vorrichtung zur Destillation oder Strippung des mindestens einen Nebenstroms aus Schritt (III). Schritt (V) kann auch mehrfach vorkommen. Als Beispiel sei der Fall genannt, dass die Aufarbeitung von Nebenströmen aus Schritt (III) auf mehrere Anlagenteile verteilt ist. Dies kann insbesondere dann der Fall sein, wenn ein Anlagenteil für die Aufarbeitung eines flüssigen Nebenstroms und ein Anlagenteil für die Aufarbeitung eines gasförmigen Nebenstroms vorgesehen ist. In diesem beispielhaft genannten Fall umfasst das Verfahren zwei Schritte (und Anlagenteile) der Kategorie (V), welche parallel nebeneinander durchgeführt werden, nämlich die Aufarbeitung des flüssigen Nebenstroms in einer weiteren Aufarbeitungseinrichtung (Schritt (Va)) und die Aufarbeitung des gasförmigen Nebenstroms in einer weiteren Aufarbeitungseinrichtung (Schritt (Vb)).

Es versteht sich von selbst, dass die Anlagenteile neben den zuvor explizit aufgeführten Apparaten auch Peripheriegeräte wie etwa Pumpen, Wärmeaustauscher und dergleichen mehr umfassen können.

Die Kreislauffahrweise kann über mehrere Apparate eines Anlagenteils eingestellt werden. Beispielsweise kann der Ausgangsstrom des letzten Apparats mehrerer in Serie geschalteter Apparate eines Anlagenteils als Eingangsstrom des ersten Apparats der in Serie geschalteten Apparate dieses Anlagenteils verwendet werden. Es ist auch möglich, die Kreislauffahrweise nur auf einen Teil der Apparate eines Anlagenteils anzuwenden, z. B. wenn der Ausgangsstrom des letzten Apparats mehrerer in Serie geschalteter Apparate eines Anlagenteils nicht in den ersten, sondern in einen weiteren Apparat dieses Anlagenteils zurückgeführt wird.

Die Kreislauffahrweise kann auch über mehrere Anlagenteile eingestellt werden. Beispielsweise kann der Ausgangsstrom des letzten Apparats eines Anlagenteils, z. B. eines Anlagenteils aus dem Reinigungsschritt (III), als Eingangsstrom des ersten Apparats eines davor liegenden Anlagenteils, z. B. eines Anlagenteils aus dem Aufarbeitungsschritt (II), verwendet werden, wobei die Kreislauffahrweise hergestellt wird, indem der Ausgangsstrom des beispielhaft genannten Aufarbeitungsschrittes (II) als Eingangsstrom des Reinigungsschrittes (III) dient.

Die Schritte (I), (II) und (III) des erfindungsgemäßen Verfahrens werden vorzugsweise im Rahmen eines kontinuierlichen Prozesses durchgeführt. Bevorzugt werden auch die Schritte (IV) und (V) im Rahmen eines kontinuierlichen Prozesses durchgeführt.

Durch das Abstellen von m₁ (und ggf. von m₂; bei *n* Substraten *i* bevorzugt durch das Abstellen aller *n* Massenströme m*ᵢ*), also dem Massenstrom des mindestens einen Substrates (der Substrate) in den Reaktor aus Schritt (I), wird sichergestellt, dass während der Unterbrechung, die, wie oben beschrieben, zum Zwecke der Revision, Wartung, Reparatur und/oder Reinigung eines Teiles der Herstellungsanlage durchgeführt wird oder durch einen Mangel an Rohstoff(en) und/oder Hilfsstoffe(n) verursacht wurde, die Reaktion in Schritt (I) nicht weiter stattfindet. Dadurch und durch die Verwendung des Ausgangsstromes mindestens eines nicht unterbrochenen Schrittes und zugehörigen Anlagenteils als Eingangsstrom des jeweiligen Schrittes und zugehörigen Anlagenteils (oder eines davor liegenden Schrittes bzw. Anlagenteils) wird sichergestellt, dass diese Schritte und zugehörige Anlagenteile jeweils "im Kreis" gefahren werden.

Dazu ist es vorgesehen, dass das erfmdungsgemäße Verfahren den Schritt (III) umfasst. Dadurch kommen die erfindungsgemäßen Effekte besonders zum Tragen. In einer bevorzugten Ausführungsform umfasst das erfindungsgemäße Verfahren auch die Schritte (IV) und (V).

In einer weiteren bevorzugten Ausführungsform der vorliegenden Erfindung wird bei einer Außerbetriebnahme eines Anlagenteils aus mindestens einem der Schritte (I) bis (V), insofern diese durchgeführt werden, in jedem nicht außer Betrieb genommenen Anlagenteil der Ausgangsstrom wieder als Eingangsstrom dieses oder eines davor liegenden Anlagenteils verwendet.

Die Vorteile des erfindungsgemäßen Verfahrens ergeben sich auch für mehrstufige Verfahren, in denen in aufeinanderfolgenden Anlagenteilen aufeinander aufbauende Reaktionen durchgeführt werden, um so zu einem dadurch aufgebauten Reaktionsprodukt zu gelangen. So umfasst das erfindungsgemäße Verfahren in einer weiteren bevorzugten Ausführungsform einen weiteren Schritt (Ia), in welchem mindestens ein drittes Substrat mit dem Produkt der Reaktion aus Schritt (I) in demselben oder in einem weiteren Reaktor zu Reaktion gebracht wird und das mindestens eine dritte Substrat mit einem Massenstrom m₃ in den Reaktor aus Schritt (Ia) eingetragen wird. Dabei sind im Rahmen der vorliegenden Erfindung auch Verfahren möglich, in denen eine Vielzahl von Reaktionen in aufeinander folgenden Reaktoren durchgeführt werden, wie beispielsweise drei, vier, fünf, sechs oder mehr. Somit sind auch Kaskadenreaktionen, Tandemreaktionen oder Dominoreaktionsführungen in einem Reaktor oder in aufeinanderfolgenden Reaktoren im Rahmen der vorliegenden Erfindung möglich und durch die Effizienz im Aufbau von chemischen Verbindungen auch vorteilhaft und daher bevorzugt.

Es ist insbesondere bevorzugt, dass bei der Abstellung der Zufuhr des mindestens einen Substrats, oder optional aller Substrate, die Zufuhr von Lösungsmittel, wenn es sich um eine Reaktion in der Flüssigphase handelt, nicht unterbrochen wird. Damit kann vorteilhafterweise erreicht werden, dass zum einen gegebenenfalls die Bildung von Nebenprodukten verhindert werden kann und zum anderen beispielsweise ein Verklumpen des Reaktionsgemisches unterbleibt. Damit kann effizient ein Verunreinigen des gewünschten Produktes, ein Verstopfen von Anlagenequipment wie beispielsweise von Rohrleitungen, Ventilen und Pumpen und das Erzeugen von nicht spezifikationsgerechter Ware vermieden werden.

Des Weiteren ist es insbesondere bevorzugt, dass bei der Abstellung der Zufuhr des mindestens einen Substrats, oder optional aller Substrate, die Zufuhr von Inertgas, wenn es sich um eine Reaktion in der Gasphase handelt, nicht unterbrochen wird. Damit kann vorteilhafterweise erreicht werden, dass zum einen gegebenenfalls die Bildung von Nebenprodukten verhindert werden kann und zum anderen beispielsweise ein Verstopfen von Anlagenequipment wie beispielsweise von Rohrleitungen, Ventilen und Pumpen und das Erzeugen von nicht spezifikationsgerechter Ware vermieden werden.

Das chemische Produkt des erfindungsgemäßen Verfahrens kann vorzugsweise ein Polycarbonat oder eines seiner Vorprodukte, ein Isocyanat oder eines seiner Vorprodukte, ein pharmazeutischer Wirkstoff, ein Olefin, Aromat, Polyolefin, oder dergleichen sein.

Beispielhaft seien hier als Di- und Polyisocyanate aromatische Di- und Polyisocyanate, wie z. B. Methylendiphenyldiisocyanat (mMDI) als Isomere oder als Isomerengemisch, Polymethylenpolyphenylpolyisocyanat (pMDI), Gemische aus Methylendiphenyldiisocyanat und Polymethylenpolyphenylpolyisocyanat (MDI), Toluylendiisocyanat (TDI) als reine Isomere oder Isomerengemisch, Isomere des Xylylendiisocyanats (XDI), Isomere des Diisocyanatobcnzols, 2,6-Xylolisocyanat, 1,5-Naphthylendiisocyanat (1,5-NDI), Diisocyanate auf Basis von aliphatischen oder cycloaliphatischen Kohlenwasserstoffen mit 2 bis 18 Kohlenstoffatomen, wie z.B. 1,4-Butandiisocyanat, 1,5-Pentandiisocyanat, 1,6-Hexandiisocyanat (HDI), 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat (TMDI), 1,3- und 1,4-Cyclohexandiisocyanat, 1-Isocyanato-3,3,5-trimethyl-5-isocyanatomethylcyclohexan (IPDI), 2,4-, oder 2,6-Diisocyanato-l-methylcyclohexan (H6-TDI), 1-Isocyanato-l-methyl-4(3)-isocyanatomethylcyclohexan (AMCI), 1,3(und/oder 1,4)-Bis(isocyanatomethyl)cyclohexan, Bis(isocyanatomethyl)norbornan (NBDI), 4,4'(und/oder 2,4')-Diisocyanatodicyclohexyl-methan, und (cyclo)aliphatische Triisocyanate mit bis zu 22 Kohlenstoffatomen, wie z.B. Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan oder 1,3,5-Tris(isocyanatomethy1)-cyclohexan genannt.

Die korrespondierenden Amine zu den obigen Polyisocyanaten sind aromatische Di- und Polyamine, wie z.B. Methylendiphenyldiamin (mMDA) als Isomere oder als Isomerengemisch, Polymethylenpolyphenylpolyamin (pMDA), Gemische aus Methylendiphenyldiamin und Polymethylenpolyphenylpolyamin (MDA), Toluylcndiamin (TDA) als reine Isomere oder Isomerengemisch, Isomere des Xylylendiamins (XDA), Isomere des Diaminobenzols, 2,6-Xylidin, 1,5-Naphthylendiamin (1,5-NDA), Diamine auf Basis von aliphatischen oder cycloaliphatischen Kohlenwasserstoffen mit 2 bis 18 Kohlenstoffatomen, wie z.B. 1,4-Diaminobutan, 1,5-Diaminopentan, 1,6-Diaminohexan (HDA), 1,8-Diaminooctan, 1,9-Diaminononan, 1,10-Diaminodecan, 2,2-Dimethyl-1,5-diaminopentan, 2-Methyl-1,5-pentandiamin (MPDA), 2,4,4(oder -2,2,4)-Trimethyl-1,6-diaminohexan (TMDA), 1,3- und 1,4-Diaminocyclohexan, 1-Amino-3,3,5-trimethyl-5-aminomethylcyclohexan (IPDA), 2,4-, oder 2,6-Diamino-1-methylcyclohexan (H6-TDA), 1-Amino-1-methyl-4(3)-aminomethylcyclohexan (AMCA), 1,3(und/oder 1,4)-Bis(aminomethyl)cyclohexan, Bis(aminomethyl)norbornan (NBDA), 4,4'(und/oder 2,4')-Diaminodicyclohexyl-methan, (cyclo)aliphatische Triamine mit bis zu 22 Kohlenstoffatomen, wie z.B. Triaminocyclohexan, Tris(aminomethyl)-cyclohexan, Triamino-methylcyclohexan, 1,8-Diamino-4-(aminomethyl)octan, 1,6,11-Undecantriamin, 1,7-Diamino-4-(3-aminopropyl)heptan, 1,6-Diamino-3-(aminomethyl)-hexan oder 1,3,5-Tris(aminomethyl)-cyclohexan.

Die großtechnische Herstellung der oben angeführten Polyisocyanate durch Umsetzung der korrespondierenden Amine mit Phosgen ist seit längerem aus dem Stand der Technik bekannt, wobei die Reaktion in der Gas- oder Flüssigphase sowie diskontinuierlich oder kontinuierlich durchgeführt werden (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise G. Wegener et. al. Applied Catalysis A: General 221 (2001), p. 303 - 335, Elsevier Science B.V. sowie Ullmanns Encyklopädie der technischen Chemie, 4. Aufl. (1977), Band 13, S. 351 bis 353.

MDI und TDI sind bezüglich der Herstellungsvolumina weltweit die größten Polyisocyanat-Produkte.

Die moderne großtechnische Herstellung von MDI erfolgt kontinuierlich, und die Reaktionsführung erfolgt als adiabate Phosgenierung, wie in EP 1 616 857 B2 und EP 1 873 142 B1 beschrieben. Die Aufarbeitung des Roh-MDI ist beispielhaft in US 5 136 087 (B), EP 1 854 783 A2, EP 1 475 367 B1 oder auch in EP 1 686 112 A1 beschrieben.

Die kontinuierliche Herstellung von TDI erfolgt großtechnisch in der Gasphase, wie z. B. in EP-A-2 196 455, EP-A-0 289 840, EP-A-0 570 799, EP-B-1 935 875 und EP-B-1 935 876 beschrieben, oder in der Flüssigphase, wie z. B. in EP 0 322 647 B1, WO 2013/139703 A1, EP 314 985 B1, EP 1 371 636 B1, EP 1 371 635 B1 und EP 1413 571 B1 beschrieben.

Die Vorstufe von MDI ist MDA. MDA wiederum wird durch Kondensation von Anilin und Formaldehyd hergestellt. Anilin wird durch Hydrierung von Nitrobenzol erhalten. Nitrobenzol wiederum entsteht durch Nitrierung von Benzol, was die pctrochcmische Basis zur Herstellung von MDI über die einzelnen Zwischenstufen darstellt.

Die kontinuierliche oder diskontinuierliche Herstellung von MDA ist z. B. in EP 1 616 890 A1, US-A-5286760, EP-A-451442 und WO-A-99/40059 offenbart.

Die kontinuierliche Herstellung von Anilin in isothermer oder adiabater Fahrweise erfolgt im großtechnischen Maßstab in der Regel durch katalytische Hydrierung von Nitrobenzol wie z. B. in GB 1 452 466 A1, EP 0011 090 A1 oder EP 0 944 578 A2 (isotherme Fahrweise) und in EP 0 696 574 B1, EP 0 696 573 B1, EP 1 882 681 A1 (adiabate Fahrweise) beschrieben. Neben den genannten Verfahren mit stationären Katalysatorbetten sind auch solche mit fluidisierten Katalysatorbetten z. B. in DE 1114820 B, DE 1133394 B oder WO 2008/034770 A1 beschrieben.

Die heute gängigen Verfahren zur großtechnischen Herstellung von Nitrobenzol entsprechen im Wesentlichen dem Konzept der adiabaten Nitrierung von Benzol durch ein Gemisch von Schwefel- und Salpetersäure, welches im Allgemeinen als Mischsäure bezeichnet wird. Ein solches Verfahren wurde erstmals in US 2,256,999 beansprucht und ist in heutigen Ausführungsformen beispielsweise in EP 0 436 443 B1, EP 0 771 783 B1 und US 6 562 247 B2 beschrieben. Auch sind isotherme Verfahren zur Nitrierung von Benzol mit Mischsäure bekannt, wie beispielsweise in EP 0 156 199 B1 beschrieben.

Die Vorstufe von TDI ist TDA. TDA wiederum wird durch Hydrierung von Dinitrotoluol (DNT) erhalten. DNT wiederum entsteht durch Nitrierung von Toluol, was die petrochemische Basis zur Herstellung von TDI über die einzelnen Zwischenstufen darstellt.

Die moderne, kontinuierliche Herstellung von TDA in isothermer und/oder adiabater Fahrweise erfolgt im großtechnischen Maßstab in der Regel durch katalytische Hydrierung von DNT wie z. B. ausführlich in WO 2011/086050 A1 und darin zitierten Literaturstellen beschrieben.

Die Herstellung des Dinitrotoluols durch Nitrierung von Toluol mit Nitriersäure (Gemisch von Salpetersäure und Schwefelsäure) war bereits Gegenstand zahlreicher Veröffentlichungen und Patentanmeldungen (Ullmanns Enzyklopedie der technischen Chemie, 4.Auflage, Band 17, Seite 391 ff, 1979, Verlag Chemie Weinheim / New York). Die industrielle Herstellung erfolgt vorwiegend isotherm mit Salpetersäure in Gegenwart von Schwefelsäure als Katalysator in kontinuierlicher Fahrweise, wie beispielsweise in H. Hermann, J. Gebauer, P. Konieczny, "Industrial Nitration of Toluene to Dinitrotoluene" in ACS-Symposium, Series 623, 234-249, 1996, ed. L.F.Albright, R.V.C Carr, R.J. Schmitt beschrieben.

Beispielhaft seien hier für die Polycarbonate Polycarbonat und deren Vorprodukte wie z. B. Bisphenol A, Bisphenol F und andere hydroxylgruppenhaltige Vorstufen genannt.

Zur Durchführung des erfindungsgemäßen Verfahrens eignet sich eine **Anlage zur Herstellung eines chemischen Produktes oder einer chemischen Zusammensetzung,** umfassend die Anlagenteile:
(I) einen Reaktor zur Ausführung der Umsetzung mindestens eines Substrats, bevorzugt mehrerer (insbesondere zweier) Substrate,
(II) einen Aufarbeitungsapparat zur Gewinnung eines Rohprodukts aus dem im Reaktor (I) erhaltenen Produktgemisch neben mindestens einem Nebenstrom enthaltend die vom Rohprodukt abgetrennten Bestandteile,
(III) einen Reinigungsapparat zur Reinigung des im Aufarbeitungsapparat (II) erhaltenen Rohprodukts zu einem gereinigten Endprodukt unter Abtrennung mindestens eines Nebenstroms enthaltend die vom gereinigten Endprodukt abgetrennten Bestandteile, sowie optional die Anlagenteile (IV) bis (V)
(IV) eine Aufarbeitungseinrichtung zur Aufarbeitung des im Aufarbeitungsapparat (II) erhaltenen mindestens einen Nebenstroms,
(V) eine Aufarbeitungseinrichtung zur Aufarbeitung des im Reinigungsapparat (III) erhaltenen mindestens einen Nebenstroms,
wobei die Anlage derart ausgestaltet ist, dass bei einer Außerbetriebnahme im Sinne wie zuvor für das erfindungsgemäße Verfahren geschildert eines oder mehrerer, jedoch nicht aller, Anlagenteile (I) bis (V), insofern diese vorhanden sind, kein weiterer Eintrag des mindestens einen Substrates in den Reaktor (I) stattfindet und unabhängig voneinander oder gleichzeitig in jedem nicht von der Außerbetriebnahme betroffenen Anlagenteil der Ausgangstrom zurückgeführt und als Eingangsstrom des jeweiligen Anlagenteils oder eines davor liegenden Anlagenteils verwendet werden kann. Die Ausgestaltung der Anlage derart, dass "*bei einer Außerbetriebnahme eines oder mehrerer Anlagenteile (I) bis (V), insofern diese vorhanden sind, kein weiterer Eintrag des mindestens einen Substrates in den Reaktor (I) stattfindet"* ist dabei so zu verstehen, dass vor oder zeitgleich mit der Außerbetriebnahme eines Anlagenteils die Substratzufuhr unterbrochen wird, d. h., dass vor oder zeitgleich mit dem Einstellen der Kreislauffahrweise in mindestens einem nicht von der Außerbetriebnahme betroffenen Anlagenteil die Substratzufuhr unterbrochen wird. **Vorrichtungstechnisch** lässt sich dies auf verschiedene Weisen realisieren, bspw. durch den Einbau prozessleittechnischer Einrichtungen, die die Substratzufuhr bei Außerbetriebnahme eines oder mehrerer Anlagenteile (bei Einstellen eines oder mehrerer Anlagenteile auf Kreislauffahrweise) automatisch unterbrechen. Die Einrichtung einer Sperrschaltung, die das Einstellen auf Kreislauffahrweise nur bei unterbrochener Substratzufuhr ermöglicht, ist ebenfalls denkbar. Geeignete Soft- und Hardware-Produkte sind kommerziell erhältlich und dem Fachmann bekannt. Ggf. nötige Programmierungs- und Anpassungsarbeiten bewegen sich im Rahmen einer für den Fachmann üblichen Routinetätigkeit. Dabei versteht sich, dass diese Anlage dazu ausgestaltet ist, darin das erfindungsgemäße Verfahren durchführen zu können. Die genannten Anlagenteile entsprechen damit den Schritten des erfindungsgemäßen Verfahrens.

In einer bevorzugten Ausführungsform der Anlage kann unabhängig voneinander oder gleichzeitig in jedem anderen nicht von der Unterbrechung betroffenen Anlagenteil der Ausgangsstrom zurückgeführt und wieder als Eingangsstrom des jeweiligen Anlagenteiles oder eines davor liegenden Anlagenteils verwendet werden.

In einer bevorzugten Ausgestaltung kann unabhängig voneinander in jedem anderen nicht von der Unterbrechung betroffenen Anlagenteil oder Reaktor der Massenausgangsstrom zurückgeführt und wieder als Masseneingangsstrom des jeweiligen Anlagenteiles oder Reaktors verwendet werden. In einer alternativen Ausgestaltung kann gleichzeitig in jedem anderen nicht von der Unterbrechung betroffenen Anlagenteil der Ausgangsstrom zurückgeführt und wieder als Eingangsstrom des jeweiligen Anlagenteiles oder eines davorliegenden Anlagenteils verwendet werden.

Bevorzugt umfasst die Anlage die Aufarbeitungseinrichtungen (IV) und (V) zur Aufarbeitung anfallender Nebenströme aus den Anlagenteilen (II) bzw. (III).

Durch das Abstellen von m₁ (und ggf. von m₂; bei *n* Substraten *i* bevorzugt durch das Abstellen aller *n* Massenströme m*ᵢ*), also des Massenstromes von mindestens einem Substrat (ggf. der Massenströme aller Substrate) in den Reaktor (I), wird sichergestellt, dass während der Unterbrechung (der Außerbetriebnahme eines oder mehrerer Anlagenteile), die, wie oben beschrieben, zum Zwecke der Revision, Reparatur, Wartung und/oder Reinigung eines Teiles der Herstellungsanlage durchgeführt wird, die Reaktion in dem Schritt (I) nicht weiter stattfindet.

Hierbei ist es insbesondere bevorzugt, dass bei der Abstellung der Zufuhr des mindestens einen Substrats, oder optional aller Substrate, die Zufuhr von Lösungsmittel, wenn es sich um eine Reaktion in der Flüssigphase handelt, nicht unterbrochen wird. Damit kann vorteilhafterweise erreicht werden, dass zum einen gegebenenfalls die Bildung von Nebenprodukten verhindert werden kann und zum anderen beispielsweise ein Verklumpen des Reaktionsgemisches unterbleibt. Damit kann effizient ein Verunreinigen des gewünschten Produktes, ein Verstopfen von Anlagenequipment wie beispielsweise von Rohrleitungen, Ventilen und Pumpen und das Erzeugen von nicht spezifikationsgerechter Ware vermieden werden.

Des Weiteren ist es insbesondere bevorzugt, dass bei der Abstellung der Zufuhr des mindestens einen Substrats, oder optional aller Substrate, die Zufuhr von Inertgas, wenn es sich um eine Reaktion in der Gasphase handelt, nicht unterbrochen wird. Damit kann vorteilhafterweise erreicht werden, dass zum einen gegebenenfalls die Bildung von Nebenprodukten verhindert werden kann und zum anderen beispielsweise ein Verstopfen von Anlagenequipment wie beispielsweise von Rohrleitungen, Ventilen und Pumpen und das Erzeugen von nicht spezifikationsgerechter Ware vermieden werden.

Sollten dabei zwei oder mehr Produktionsstraßen bzw. Reaktorlinien parallel betrieben werden, dann können zunächst in einer Produktionsstraße bzw. Reaktorlinie erfindungsgemäß ein oder mehrere Anlagenteile außer Betrieb genommen werden und die andere(n) Produktionsstraße(n) bzw. Reaktorlinie(n), insofern erforderlich, nacheinander im Hinblick auf die Außerbetriebnahme eines oder mehrerer zugehöriger Anlagenteile erfindungsgemäß betrieben werden. Alternativ ist es im Rahmen der vorliegenden Erfindung aber auch möglich, alle Produktionsstraßen bzw. Reaktorlinien, insofern erforderlich, gleichzeitig oder zeitnah gemäß dem erfindungsgemäßen Verfahren im Hinblick auf die Außerbetriebnahme eines oder mehrerer zugehöriger Anlagenteile der Anlage zu betreiben.

Ein weiterer Gegenstand der vorliegenden Erfindung ist ein **Verfahren zum Betrieb einer Anlage zur Herstellung eines chemischen Produktes oder einer Produktzusammensetzung während einer Unterbrechung des Herstellungsverfahrens,** die Anlage umfassend die Anlagenteile:
(I) einen Reaktor zur Ausführung der Umsetzung mindestens eines Substrats, bevorzugt mehrerer (insbesondere zweier) Substrate,
(II) einen Aufarbeitungsapparat zur Gewinnung eines Rohprodukts aus dem im Reaktor (I) erhaltenen Produktgemisch neben mindestens einem Nebenstrom enthaltend die vom Rohprodukt abgetrennten Bestandteile,
(III) einen Reinigungsapparat zur Reinigung des im Aufarbeitungsapparat (II) erhaltenen Rohprodukts zu einem gereinigten Endprodukt unter Abtrennung mindestens eines Nebenstroms enthaltend die vom gereinigten Endprodukt abgetrennten Bestandteile, sowie optional die Anlagenteile (IV) bis (V)
(IV) eine Aufarbeitungseinrichtung zur Aufarbeitung des im Aufarbeitungsapparat (II) erhaltenen mindestens einen Nebenstroms,
(V) eine Aufarbeitungseinrichtung zur Aufarbeitung des im Reinigungsapparat (III) erhaltenen mindestens einen Nebenstroms,
das Verfahren umfassend die Schritte
(i) Stoppen der Zufuhr des mindestens einen Substrats, bevorzugt aller Substrate in den Reaktor (I),
(ii) Fahren mindestens eines Anlagenteiles so, dass der Ausgangsstrom des jeweiligen Anlagenteils als Eingangsstrom dieses Anlagenteils oder eines davor liegenden Anlagenteils verwendet wird;
(iii) Außerbetriebnahme mindestens eines Anlagenteils, jedoch nicht aller Anlagenteile;
(iv) Optional Öffnen des in Schritt (iii) außer Betrieb genommenen mindestens einen Anlagenteils;
(v) Durchführen einer Instandsetzungs-, Reinigungs-, Revisions- und/oder Reparaturmaßnahme in dem in Schritt (iii) außer Betrieb genommenen Anlagenteil;
(vi) Optional Schließen und optional Inertisieren des mindestens einen Anlagenteils aus Schritt (v);
(vii) Inbetriebnahme des mindestens einen Anlagenteils aus Schritt (vi);
(viii) Starten der Zufuhr des mindestens einen Substrats in den Reaktor (I);
wobei in Schritt (ii) jedes nicht außer Betrieb genommene Anlagenteil so gefahren wird, dass der Ausgangsstrom des jeweiligen Anlagenteils als Eingangsstrom dieses Anlagenteils oder eines davor liegenden Anlagenteils verwendet wird.

Schritt (ii) umfasst dabei die Maßnahmen, die gemäß des Verfahrens des ersten Gegenstandes der vorliegenden Erfindung getroffen werden. Somit wird auf die obenstehenden Maßnahmen der erfindungsgemäßen Kreislauffahrweise Bezug genommen, diese werden daher an dieser Stelle nicht erneut im Detail wiederholt. Auch die zu betreibende Anlage ist vorzugsweise eine Anlage wie zuvor geschildert. Mit dem vorliegenden Verfahren lassen sich daher auch die obenstehenden Vorteile der vorliegenden Erfindung erzielen.

Auf diese Art und Weise wird ein Verfahren bereit gestellt, mit welchem höchst effizient und zeitsparend sowie unter Vermeidung von Abfällen eine Wartungs-, Revisions-, Reinigungs-, Instandsetzungs- und/oder Reparaturmaßnahme an einer Anlage zur Herstellung von chemischen Verbindungen durchgeführt werden kann.

In einer weiteren bevorzugten Ausführungsform umfasst insbesondere Schritt (ii) das Umleiten des Ausgangsstromes des jeweiligen Anlagenteils als Masseneingangsstrom des jeweiligen Anlagenteils oder eines davor liegenden Anlagenteils. Wie gemäß dem ersten Gegenstand der vorliegenden Erfindung können auch zwei Anlagenteile zusammen im Kreis gefahren werden. Damit können sowohl parallel geschaltete als auch in Serie geschaltete Anlagenteile gemeint sein.

Die beigefügten Zeichnungen sollen die Erfindung noch näher veranschaulichen:
FIG. 1 zeigt eine Produktionsanlage für ein chemisches Produkt oder eine chemische Zusammensetzung im Regelbetrieb:
   Zwei Substrate 1 und 2 werden einem Reaktor 1000 zugeführt. Das erhaltene Reaktionsgemisch 3 wird in einem Aufarbeitungsapparat 2000 in bspw. eine wässrige Phase 4, eine gasförmige Phase 5 mit bspw. gasförmigen Nebenkomponenten und in eine Produkt-haltige organische Phase 6 aufgetrennt. Dies kann beispielsweise in einem einfachen Phasentrennapparat geschehen. Strom 6 wird im Reinigungsapparat 3000 zum gewünschten Produkt 8 aufgereinigt, wobei Nebenkomponenten 7 abgetrennt werden. 3000 kann beispielsweise eine Destillationsapparatur sein, in der leichtsiedende Nebenkomponenten 7 vom Produkt 8 abgetrennt werden.
FIG. 2 zeigt eine mögliche Ausgestaltung einer erfindungsgemäßen Kreisfahrweise:
   Der Reaktor 1000 soll außer Betrieb genommen werden, um bspw. Wartungsarbeiten durchzuführen. Hierzu wird die Zufuhr der Substrate 1 und 2 unterbrochen. In der gezeigten Ausführungsform wird nun der Sumpfaustrag 8 der Destillationskolonne 3000 in den Phasentrennapparat 2000 und von dort wieder in die Destillationskolonne 3000 geführt.
FIG. 3 zeigt eine weitere mögliche Ausgestaltung einer erfindungsgemäßen Kreisfahrweise:
   Der Phasentrennapparat 2000 soll außer Betrieb genommen werden, um bspw. Wartungsarbeiten durchzuführen. Hierzu wird die Zufuhr der Substrate 1 und 2 unterbrochen. In der gezeigten Ausführungsform wird der Reaktoraustrag 3 in den Reaktor 1000 zurückgeführt. Gleichzeitig wird der Sumpfaustrag 8 der Destillationskolonne 3000 in dieselbe zurückgeführt.
FIG. 4 zeigt eine weitere mögliche Ausgestaltung einer erfindungsgemäßen Kreisfahrweise:
   Die Destillationskolonne 3000 soll außer Betrieb genommen werden, um bspw. Wartungsarbeiten durchzuführen. Hierzu wird die Zufuhr der Substrate 1 und 2 unterbrochen. In der gezeigten Ausführungsform wird der Austrag an Organika 6 des Phasentrennapparats 2000 in den Reaktor 1000 und von diesem in den Phasentrennapparat 2000 zurückgeführt. Noch in 2000 vorhandene Restmengen an wässriger Phase werden entweder ausgetragen oder verbleiben im Apparat.

Durch das erfindungsgemäße Verfahren ergeben sich die folgenden Vorteile:
i) Erhöhung der Produktivität, weil sich die Verfügbarkeit der Anlage erhöht, da sich der Zeitbedarf für das Abfahren und Wiederanfahren der Anlage für den Produktionsstillstand stark minimiert.
ii) Investitionskosten in eine größere Anlagenkapazität entfallen.
iii) Investitionskosten in einen größeren Endprodukttank zur Abpufferung von längeren Stillstandzeiten entfallen.
iv) Vermeidung von überflüssigen Abfallprodukten, Abwässern und Abgasen, die zusätzlich aufgereinigt werden müssen. Diese entstünden, wenn die Anlage komplett neu angefahren werden müsste.
v) In vielen Fällen kommt es zur Einsparung von Energien, weil die zum Wiederanfahren benötigten Vorbereitungen für die ausgeschalteten Anlagenteile wie das Aufwärmen der Einsatz- und Hilfsstoffe oder das Aufwärmen des Equipments etc. entfallen.
vi) In vielen Fällen kommt es zur Einsparung von Hilfsstoffen wie Kondensat und Stickstoff.
vii) Die Reparaturanfälligkeit von Pumpen oder Kompressoren wird vermindert, da, wenn diese bei einem Stillstand abgestellt werden, bei jedem Wiederanfahren deren Gleitringdichtungen leiden. Somit werden Folgereparaturen vermieden, was sich wiederum positiv auf die Produktivität der Anlage und die Instandhaltungskosten auswirkt.

Dabei können die Vorteile i) bis vii) jeweils einzeln oder vorteilhafterweise in Kombination auftreten.

Der Erfolg der erfindungsgemäßen Vorgehensweise ist für den Fachmann überraschend, weil er, um grundsätzlich Energie einzusparen und um sich auf die anstehenden Instandhaltungsmaßnahmen im Produktionsstillstand konzentrieren zu können, viel eher dazu tendieren würde, *die Gesamtanlage* abzustellen, zumal für das erfindungsgemäße Verfahren bzw. für die erfindungsgemäße Anlage Zusatzinvestitionen in rückführende Rohrleitungen samt Pumpen, Umbauten an den Apparaten sowie zusätzliche Prozessleittechnik in Kauf genommen werden.

Im Folgenden soll die vorliegende Erfindung anhand von Beispielen verdeutlicht werden.

### Beispiele

Gehaltsangaben in ppm oder % sind Massenanteile bezogen auf die Gesamtmasse des jeweiligen Stoff(strom)s. Analysenwerte wurden, sofern nicht anders angegeben, mittels Gaschromatographie bestimmt.

### Allgemeine Bedingungen für die Herstellung von Nitrobenzol bei einer eingefahrener Produktionsanlage

In einen Nitrierreaktor wurden bei einer Produktionslast von 50 t/h Nitrobenzol ein Schwefelsäure-, ein Salpetersäure-, ein Frischbenzol- und ein Rückbenzolstrom eindosiert. Es wurde ein 5 bis 10 %iger Überschuss an Benzol, bezogen auf Salpetersäure, eingesetzt. Nach vollständiger Umsetzung der Salpetersäure mit dem Benzol zu Nitrobenzol unter adiabater Reaktionsführung wurde das nunmehr ca. 130 °C heiße Reaktionsprodukt einem Phasentrennapparat zugeführt, in dem das Reaktionsprodukt in eine organische Phase (= Roh-Nitrobenzol, enthaltend neben Nitrobenzol noch Benzol) und eine wässrige Phase (= Absäure, enthaltend neben Schwefelsäure noch geringe Anteile an Nitrobenzol und Benzol) zerfiel. Die wässrige, hauptsächlich Schwefelsäure umfassende Phase wurde im Verdampfer durch plötzliche Druckerniedrigung einer Blitzverdampfung von Wasser unterzogen und auf diese Weise aufkonzentriert. Die aufkonzentrierte Schwefelsäure wurde im Schwefelsäuretank zur erneuten Verwendung gelagert. Nach dem Abtrennen im Phasentrennapparat wurde das Rohnitrobenzol in der Rohnitrobcnzolkühlung auf ca. 50 °C abgekühlt und der Wäsche zugeführt. Der so erhaltene von Nitrophenolen und Salzen weitgehend befreite Strom gereinigten Roh-Nitrobenzols wurde wieder aufgeheizt und in einer Destillationskolonne von Wasser, Benzol und anderen Leichtsiedern, welche über Kopf abgetrennt wurden, befreit, wodurch getrocknetes Rein-Nitrobenzol erhalten wurde. Das kondensierte Kopfprodukt der Destillationskolonne wurde einem Phasentrennapparat zugeführt, in dem das Kopfprodukt in eine organische Phase (enthaltend Benzol) und eine wässrige Phase zerfiel. Die organische Phase wurde in einem Puffertank zwischengelagert und von dort, wie oben schon beschrieben, zur Reaktion in den Zulauf des Nitrierreaktors zurückgefahren. Der Stromverbrauch einer derartigen Anlage liegt bei ca. 890 kW/h.

Auf diese Weise hergestelltes Nitrobenzol hat typischerweise eine Reinheit von ca. 99,96 % (GC), einen Restbenzolgehalt von 0,0028 % (GC), einen Gehalt an 1,3-Dinitrobenzol von 0,0273 % (GC) und einen Nitrophenolgehalt von < 5 ppm (HPLC). Ferner weist das Nitrobenzol einen Wassergehalt von 0,0079 % (bestimmt nach Karl-Fischer) auf.

**Vergleichsbeispiel** 1: Kurzstillstand einer Produktionsanlage mit Komplettabstellung der Anlage, Reinigungsmaßnahme und Wiederanstellung der Anlage.

Der Kurzstillstand der Anlage diente dazu, Reinigungsarbeiten im Nitrierbereich durchzuführen. Hierfür wurde die Anlage komplett abgefahren, also Nitrierung, Wäschen, Destillation, Sauerwasseraufarbeitung und alkalische Abwasseraufarbeitung. Die Energiezufuhren während den Reinigungsarbeiten wurden abgestellt. Nach den Reinigungsarbeiten wurde wieder angefahren, wobei die komplette Anlage vorab inertisiert, befüllt und aufgeheizt werden musste.

### Durchführung der Komplettabstellung der Anlage:

Als erstes wurde die Nitrierung abgestellt: Die Dosierpumpen der Eingangsströme von Benzol und Salpetersäure wurden abgestellt. Der Dampf des Blitzverdampfers wurde 5 Minuten nach den Rohstoffen Benzol und Salpetersäure abgestellt. Die Kreislaufschwefelsäure lief 1 Stunde weiter, bis alle Organika aus dem Nitrierkreislauf, bestehend aus Nitratoren, Phasentrennapparat, Blitzverdampfer und Kreislaufschwefelsäurevorlagetank, ausgetragen worden waren. Dann wurde der 100 °C heiße Schwefelsäurekreislauf durch Abstellen der Kreislaufpumpe unterbrochen. Die Nitratoren, der Phasentrennapparat und der Blitzverdampfer blieben unter Schwefelsäure stehen. Die restliche Kreislaufschwefelsäure befand sich im Schwefelsäurevorlagetank. Das Gesamtinventar an Schwefelsäure betrug 74 Tonnen. Gleichzeitig mit der Kreislaufpumpe wurde die Vakuumpumpe zum Blitzverdampfer abgestellt und das Vakuum mit Stickstoff aufgehoben. Nun stand der Nitrierkreislauf. Zeitbedarf 2 Stunden, ohne Vakuum aufheben 1 Stunde.

Danach wurde die Sauerwasseraufarbeitung abgestellt, indem die Einspeisung des sauren Abwassers aus dem Sauerwasservorlagetank zum Sauerwasserstripper unterbrochen wurde. Der Dampf zum Sauerwasserstripper und die Sumpfpumpe des Strippers wurden abgestellt. Nun stand die Sauerwasseraufarbeitung. Der Zeitbedarf war 5 Minuten.

Als nächstes wurden die Wäschen abgestellt, indem die Rohnitrobenzolzufuhr aus dem Rohnitrobenzoltank zur sauren Wäsche unterbrochen wurde. Der Rohnitrobenzolweg durch die saure, alkalische und neutrale Wäsche wurde abgestellt, indem die Förderpumpen des Rohnitrobenzols vor den jeweiligen Wäschen gestoppt wurden. Die Wäschen wiesen eine Betriebstemperatur von 48 °C auf und blieben mit Rohnitrobenzol gefüllt. Gleichzeitig wurden der saure, der alkalische und der neutrale Waschwasserweg durch Abschalten der jeweiligen Pumpen abgestellt. Der Zeitbedarf war 5 Minuten.

Dann wurde die Destillation abgestellt, indem die Einspeisung von Rohnitrobenzol unterbrochen wurde und der Dampf zur Destillationskolonne weggenommen wurde. Unmittelbar danach wurde der Produktaustrag durch Abstellen der Sumpfpumpe unterbrochen und der Rücklauf am Kolonnenkopf durch Stoppen der Benzolpumpe abgestellt. Nachdem die Vakuumpumpe abgestellt und mit Stickstoff belüftet war, stand die Destillation. Der Zeitbedarf war 5 Minuten.

Als letztes wurde die alkalische Abwasseraufarbeitung abgestellt, indem die thermische Druckzersetzung auf Kreislauffahrweise gestellt wurde und der Dampf zur Druckzersetzung abgestellt wurde. Gleichzeitig wurden die Zufuhr und der Ablauf des alkalischen Abwassers des Strippers durch Ausschalten der Abwasserpumpen gestoppt und der Dampf zum Stripper geschlossen. Der Zeitbedarf war 5 Minuten. Die Kreislauffahrweise der thermischen Druckzersetzung (TDZ) wurde nach 10 Stunden gestoppt, sobald das Kreislaufwasser unter 100 °C abgekühlt war.

Die Komplettabstellung dauerte ohne Spülung und Entleerung der Apparate, Pumpen und Rohrleitungen insgesamt zwei Stunden, wenn man vom Abfahren (Kaltfahren) der TDZ absieht. In modernen, automatisierten Produktionsanlagen spielt die Personalstärke für den Abfahrprozess nur eine untergeordnete Rolle.

### Durchführung der Reinigungsmaßnahme:

Es war eine Verstopfung im Benzolvorwärmer im Ablauf des Rohnitrobenzols vom Phasentrennapparat zum Rohnitrotank zu beseitigen:
Zuerst wurde der Stand im Phasentrennbehälter um 50 % abgesenkt, damit keine Organik in den zu reinigenden Benzolvorwärmer nachlaufen konnte. Anschließend wurde der Benzolvorwärmer über einen Spülstutzen, der zwischen Phasentrennapparat und Benzolvorwärmer angebracht wurde, für 1 Stunde mit Kondensat gespült, um Rohnitrobenzol und Spuren an Schwefelsäure zu entfernen. Das Spülkondensat wurde zur sauren Wäsche abgeleitet. Danach wurde der Benzolvorwärmer mechanisch von Zu- und Ablauf getrennt und schwarze Niederschläge im Benzolvorwärmer, die die Verstopfung ausmachten, wurden mit großen Mengen Kondensat mit zwei weiteren Spülstutzen zur Kläranlage herausgespült. Der Zeitbedarf lag bei 3 Stunden. Nach Demontage aller 3 Spülstutzen wurden der Zu- und Ablauf des Benzolvorwärmers montiert. Der Zeitbedarf hierfür war 2 Stunden. Danach wurden die betroffenen Rohrleitungen mehrfach aufgewärmt und abgekühlt und dabei die Flanschverbindungen mit den neuen Dichtungen nachgezogen. Der Zeitbedarf betrug 2 Stunden.

Die Reinigungsmaßnahme dauerte insgesamt 8 Stunden. In modernen, automatisierten Produktionsanlagen spielt die Personalstärke für die Vorbereitung der Reinigungsmaßnahme, nämlich Teilentleerung der Anlage, Montage des Spülstutzens zur Vorreinigung des Benzolvorwärmers mit Zu- und Ablauf und anschließender Vorreinigung mit Kondensat, eine wichtige Rolle. In diesem Fall wird ein zusätzlicher Produktionsmitarbeiter benötigt. Handwerker zur Demontage und Montage der Rohrleitungen für die Reinigungsmaßnahme und das Reinigungspersonal selbst werden ebenfalls benötigt.

### Durchführung der Wiederanstellung der Anlage:

Vorab wurden in der gesamten Produktionsanlage die Vakuumpumpen in Betrieb genommen. Der Phasentrennapparat und der gereinigte Benzolvorwärmer wurden mit 100 Nm³ Stickstoff inertisiert.

Als erstes wurden die Wäschen angestellt, indem durch Anstellen der Rohnitrobenzolpumpe die Rohnitrobenzolzufuhr aus dem Rohnitrobenzoltank zur sauren Wäsche in Betrieb genommen wurde. Danach wurden der saure, der alkalische und der neutrale Waschwasserweg durch Einschalten der jeweiligen Pumpen angestellt. Dann wurde der Rohnitrobenzolweg durch die saure, alkalische und neutrale Wäsche angestellt, indem die Förderpumpen des Rohnitrobenzols vor den jeweiligen Wäschen eingeschaltet wurden. Die Waschapparate, die mit Rohnitrobenzol und Waschwasser gefüllt waren, hatten 45 °C und wärmten sich nach dem Anstellen der Produktionsanlage langsam wieder auf 48 °C auf.

Nachdem die letzte Stufe der neutralen Wäsche durch Einspeisen von 3 t/h Kondensat in Betrieb genommen war, wurde die Destillation angestellt, indem Vakuum auf die Destillationskolonne gegeben und aus der letzten neutralen Wäsche 45 °C warmes Rohnitrobenzol zur Destillationskolonne gefahren wurden. Danach wurde die Sumpfpumpe der Kolonne angestellt und Rohnitrobenzol zum Rohnitrobenzoltank gefahren. Nun wurde die Destillationskolonne mit 2 t/h 16 bar Dampf beaufschlagt und auf 170 °C aufgeheizt. Bei 50 °C im Kopf der Kolonne wurde der Rücklauf durch Anstellen der Benzolpumpe in Betrieb genommen. Die Wäschen und die Destillation waren nach 4,5 Stunden zum Wiederanfahren der Produktionsanlage bereit.

Parallel zu den Wäschen und der Destillation wurde die Sauerwasseraufarbeitung angestellt, indem 1 t/h 6 bar Dampf zum Aufwärmen des Sauerwasserstrippers aufgegeben wurden und die Sumpfpumpe des Strippers angestellt wurde. Anschließend wurde die Einspeisung des sauren Abwassers aus dem Sauerwasservorlagetank zum Sauerwasserstripper angestellt. Nun stand die Sauerwasseraufarbeitung zum Wiederanfahren der Produktionsanlage bereit. Der Zeitbedarf zum Anstellen des Sauerwasserstrippers inklusive der Analytik des sauren Abwassers auf Organika mittels Gaschromatographie war 1 Stunde.

Parallel zu den Wäschen und der Destillation wurde die alkalische Abwasseraufarbeitung angestellt, indem die in Kreislauffahrweise gestellte thermische Druckzersetzung mit 0,6 t/h 110 bar Dampf beaufschlagt wurde, um das Kreislaufwasser von 85 °C auf 285 °C zu bringen. 2 Stunden vor dem Ausschleusen des alkalischen Abwassers wurde der Stripper mit 0,5 t/h 6 bar Dampf beaufschlagt und die Zufuhr und der Ablauf des alkalischen Abwassers des Strippers wurden durch Anschalten der Abwasserpumpen angestellt. Der Zeitbedarf war 8 Stunden.

Eineinhalb Stunden, bevor die Wäschen und die Destillation zum Wiederanfahren der Produktionsanlage bereit waren und nachdem die Sauerwasseraufarbeitung in Kreislauffahrweise lief, wurde die Schwefelsäurekreislaufpupe angestellt und die Schwefelsäure über den Nitrator, Phasentrennapparat, Blitzverdampfer und Schwefelsäurevorlagetank im Kreis gefahren. Im Blitzverdampfer wurde das Vakuum angestellt und anschließend 2,4 t/h 6 bar Dampf aufgegeben, womit die Kreislaufschwefelsäure auf Starttemperatur erhitzt wurde. Dieser Vorgang dauerte 1 Stunde, bis die auf 93 °C abgekühlte Kreislaufschwefelsäure auf 100 °C aufgewärmt war.

Nach 4,5 Stunden waren die Wäschen und die Destillation betriebsbereit und die Produktionsanlage wurde durch Anstellen der Benzol- und Salpetersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an und der Sauerwasserstripper wurde auf Ausschleusen des sauren Abwassers gestellt und die Sumpfkolonne der Destillation wurde auf Produktaustrag des Endproduktes Nitrobenzol gestellt. Das Hochziehen der Produktionsanlage auf Nennlast, das in einer modernen Produktionsanlage automatisiert ist, dauerte nochmals 1 Stunde.

### Bilanz des Energie-, Hilfsstoff- und Zeitbedarfes für das Ab- und Anfahren der Anlage inklusive der Reinigungsmaßnahme:

Der gesamte Zeitbedarf für die Maßnahme betrug 15 Stunden. Dies gilt für den Fall, wenn genügend Personal vorhanden ist und keine technischen Schwierigkeiten auftreten. Der Zeitbedarf für die Reinigung an sich betrug 8 Stunden. Für das Abfahren wurden 2,5 Stunden benötigt. Das Anfahren dauerte 4,5 Stunden.

Insgesamt gingen so 775 Tonnen an Produktion von Nitrobenzol verloren. Der Dampfverbrauch betrug 4,4 Tonnen 6 bar Dampf, 8 Tonnen 16 bar Dampf und 4,8 Tonnen 110 bar Dampf. Beim Abfahren der Anlage und während der Maßnahme wurde kein Dampf verbraucht. Der Verbrauch an Stickstoff betrug für das Abfahren 550 Nm³ und für das Wiederanfahren der Anlage 100 Nm³. Der Verbrauch an Kondensat betrug 15,5 m³ (2 m³ für das Spülen des Wärmetauschers und 13,5 m³ für das Anfahren der neutralen Wäsche). Der Verbrauch an Strom betrug insgesamt 6130 kW. Für das Abfahren der Anlage wurden 1100 kW für die TDZ, 180 kW für die Nitrierung und 445 kW für die Wäschen benötigt. Für das Anfahren der Anlage wurden 4005 kW für die Wäschen und 400 kW für die Kreislaufschwefelsäurepumpen benötigt. Während der Reinigungsmaßnahme wurde kein Strom verbraucht.

**Beispiel 1:** Kurzstillstand der Produktionsanlage mit Kreislauffahrweise der nicht von der Reinigungsmaßnahme betroffenen Anlagenteile, Reinigungsmaßnahme und Wiederanstellung der Anlage.

Der Kurzstillstand der Anlage diente für Reinigungsarbeiten im Nitrierbereich. Dazu wurde der Nitrierbereich komplett abgefahren und die anderen Anlagenteile wie die Wäschen, die Destillation, die saure und die alkalische Abwasseraufarbeitung in Kreislauffahrweise gestellt. Die Energien während der Reinigungsarbeiten waren nur im Nitrierbereich abgestellt (Das Vakuum verblieb in stand-by). Nach den Reinigungsarbeiten wurde wieder angefahren, wobei nur der Nitrierbereich komplett inertisiert, befüllt und aufgeheizt werden musste.

### Durchführung der Komplettabstellung der Nitrierung und Einstellung der restlichen Anlagenteile auf Kreislauffahrweise:

Als erstes wurde die Sauerwasseraufarbeitung in Kreislauffahrweise gestellt, indem die Ausschleusung des sauren Abwassers in den Abwasserkanal mittels der Sumpfpumpe des Strippers, die 10 kW/h benötigt, zurück in den Sauerwasservorlagetank gestellt wurde. Der Dampf zum Sauerwasserstripper wurde von 1,2 t/h auf 0,7 t/h 6 bar Dampf gedrosselt und die Einspeisung von Sauerwasser aus dem Sauerwasservorlagetank mittels der Sauerwasserpumpe, die 10 kW/h benötigt, in den Sauerwasserstripper wurde von 20 m³ auf 13 m³ reduziert. Das Umstellen der Sauerwasseraufarbeitung auf Kreislauffahrweise erfolgte mittels Automatik in 33 Sekunden.

Als nächstes wurde die alkalische Abwasseraufarbeitung in Kreislauffahrweise gestellt, indem die Ausschleusung des alkalischen Abwassers der thermische Druckzersetzung (TDZ) in den Abwasserkanal mittels der Hochdruckpumpe der TDZ, die 55 kW/h benötigt, zurück in die alkalische Abwasservorlage gestellt wurde. Der Dampf zur Druckzersetzung wurde von 0,32 t/h auf 0,20 t/h 110 bar Dampf gedrosselt und die Einspeisung von alkalischem Abwasser aus der alkalischen Abwasservorlage in die TDZ wurde von 4,0 m³/h auf 2,5 m³/h reduziert. Das Umstellen der TDZ auf Kreislauffahrweise erfolgte mittels Automatik in 5 Minuten, weil die Reduzierung der Einspeisung zur TDZ manuell erfolgte.

Gleichzeitig wurde der Stripper des alkalischen Abwassers in Kreislauffahrweise gestellt, indem die Ausschleusung des alkalischen Abwassers in die TDZ unterbrochen wurde und mittels der Sumpfpumpe des Strippers, die 10 kW/h benötigt, das alkalische Abwasser zurück auf den Abwassertank gestellt wurde. Der Dampf zum Stripper des alkalischen Abwassers wurde von 0,4 t/h auf 0,25 t/h 6 bar Dampf gedrosselt und die Einspeisung von alkalischem Abwasser aus dem Abwassertank in den Stripper des alkalischen Abwassers wurde mittels der Einspritzpumpe des alkalischen Abwassers, die 10 kW/h benötigt, von 4 m³ auf 2,5 m³ reduziert. Das Umstellen des Strippers des alkalischen Abwassers auf Kreislauffahrweise erfolgte mittels Automatik in 27 Sekunden.

Als nächstes wurden die Wäschen und die Destillation in Kreislauffahrweise gestellt, indem die Ausschleusung des Endproduktes Nitrobenzol aus der Nitrobenzol-Kolonne zum Nitrobenzollagertank mittels der Sumpfpumpe der Kolonne, die 24 kW/h benötigt, auf den Rohnitrobenzoltank umgestellt wurde. Gleichzeitig wurde die Benzol enthaltende organische Phase des Brüdenphasentrennapparates über natürlichen Ablauf zum Rohnitrobenzoltank geführt. Die wässrige Phase des Brüdenphasentrennapparates wurde über die saure Wäsche und die saure Abwasseraufarbeitung entsorgt. Das Vakuumsystem der Nitrobenzol-Kolonne blieb in Betrieb. Die Kreislauffahrweise wurde hergestellt, indem der Inhalt des Rohnitrobenzoltanks mittels Förderpumpen über die gesamten Wäschen zurück zur Nitrobenzol-Kolonne gefahren wurde. Der Rohnitrotank, die saure Wäsche, die alkalische Wäsche und die 3 neutralen Wäschen haben jeweils eine Förderpumpe, die jeweils 24 kW/h benötigen. Die Einspeisung von Rohnitrobenzol in die Wäschen bzw. die Destillation wurde von 42 t/h auf 27 t/h reduziert. Der Dampf zur Nitrobenzol-Kolonne wurde von 2,5 t/h auf 1,6 t/h 16 bar Dampf gedrosselt. Das Waschwasser zur neutralen Wäsche wurde von 6,3 m³/h auf 4,0 m³/h reduziert. Das Umstellen der Wäschen und der Destillation auf Kreislauffahrweise erfolgte mittels Automatik in 5 Minuten,

Als letztes wurde die Nitrierung abgestellt, indem die Dosierpumpen der Eingangsströme von Benzol und Salpetersäure abgestellt wurden. Der Dampf des Blitzverdampfers wurde 5 Minuten nach den Rohstoffen Benzol und Salpetersäure abgestellt. Die Kreislaufschwefelsäure lief 1 Stunde weiter, bis alle Organika aus dem Nitrierkreislauf, bestehend aus Nitratoren, Phasentrennapparat, Blitzverdampfer und Kreislaufschwefelsäurevorlagetank, ausgetragen waren. Dann wurde der 100 °C heiße Schwefelsäurekreislauf durch Abstellen der Kreislaufpumpe unterbrochen. Die Nitratoren, der Phasentrennapparat und der Blitzverdampfer blieben unter Schwefelsäure stehen. Die restliche Kreislaufschwefelsäure befand sich im Schwefelsäurevorlagetank. Gleichzeitig mit der Kreislaufpumpe wurde die Vakuumpumpe zum Blitzverdampfer abgestellt und das Vakuum mit 350 Nm³ Stickstoff aufgehoben. Nun stand der Nitrierkreislauf. Der Zeitbedarf für die Abstellung betrug 2 Stunden.

Die Vorbereitung (Einstellen Kreislauffahrweise von Wäsche, Destillation, alkalisches und saures Abwasser und das Abstellen der Nitrierung) für die Reinigungsmaßnahme dauerte ohne Spülung und Entleerung der Apparate, Pumpen und Rohrleitungen insgesamt 2 Stunden und 11 Minuten.

**Durchführung der Reinigungsmaßnahme:** die Reinigungsmaßnahme wurde, wie in Beispiel 1 beschrieben, durchgeführt.

### Durchführung der Wiederanstellung der Anlage:

Vorab wurde die Vakuumpumpe für den Blitzverdampfer in Betrieb genommen. Der Phasentrennapparat und der gereinigte Benzolvorwärmer wurden mit 100 Nm³ Stickstoff inertisiert. Die Inbetriebnahme der Anlage wurde mit dem Anfahren der Nitrierung gestartet, indem die Schwefelsäurekreislaufpumpe angestellt und die Schwefelsäure über den Nitrator, Phasentrennapparat, Blitzverdampfer und Schwefelsäurevorlagetank im Kreis gefahren wurde. Im Blitzverdampfer war das Vakuum bereits angestellt und es wurden 2,4 t/h 6 bar Dampf aufgegeben, womit die Kreislaufschwefelsäure auf Starttemperatur erhitzt wurde. Dieser Vorgang dauerte 1 Stunde, bis die auf 93 °C abgekühlte Kreislaufschwefelsäure auf 100 °C aufgewärmt war. Nun wurde die Nitrierung durch Anstellen der Benzol- und Salpetersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an, der Sauerwasserstripper wurde auf Ausschleusen des sauren Abwassers gestellt und die Sumpfkolonne der Destillation wurde auf Produktaustrag des Endproduktes Nitrobenzol gestellt. Gleichzeitig wurde der Stripper der alkalischen Abwasseraufarbeitung auf Ausschleusung zur TDZ und die TDZ von Kreislauffahrweise auf Ausschleusung in den Abwasserkanal gestellt. Das Hochziehen der Produktionsanlage auf Nennlast, das in einer modernen Produktionsanlage automatisiert ist, dauerte nochmals 1 Stunde.

### Bilanz des Energie- und Zeitbedarfes für Ab- und Anfahren aus Kreislauffahrweise der Anlage inklusive der Reinigungsmaßnahme:

Der gesamte Zeitbedarf für die Maßnahme betrug 11 Stunden und 12 Minuten. Dieses gilt insbesondere für den Fall, wenn genügend Personal vorhanden ist und keine technischen Schwierigkeiten auftreten. Der Zeitbedarf für die Reinigung an sich betrug 8 Stunden. Für das Abfahren in Kreislauffahrweise wurden 2 Stunden und 11 Minuten benötigt. Das Anfahren aus der Kreislauffahrweise dauerte 1 Stunde und 1 Minute.

Insgesamt gingen auf diese Weise 585 Tonnen an Produktion von Nitrobenzol verloren. Der Dampfverbrauch betrug 12 Tonnen 6 bar Dampf, 13 Tonnen 16 bar Dampf und 1,6 Tonnen 110 bar Dampf für die Kreislauffahrweise. Beim Abfahren der Anlage in die Kreislauffahrweise wurde kein Dampf verbraucht. Der Verbrauch an Stickstoff betrug für das Abfahren in Kreislauffahrweise 350 Nm³ und für das Wiederanfahren der Anlage aus Kreislauffahrweise 100 Nm³. Der Verbrauch an Kondensat für die Maßnahme betrug 47 m₃ (2 m₃ für das Spülen des Wärmetauschers und 45 m₃ für An- und Abfahren und Kreislauffahrweise der Wäschen). Der Verbrauch an Strom betrug insgesamt 8525 kW. Für das Abfahren der Anlage wurden 1943 kW, für die Kreislauffahrweise während der Reinigungsmaßnahme 5680 kW für die Kreislauffahrweise und für das Anfahren der Anlage 905 kW an Strom verbraucht.

### Fazit der Komplettabstellung (Vergleichsbeispiel 1) versus Kreislauffahrweise (Beispiel 1) für die Reinigungsmaßnahme:

Als Fazit der Komplettabstellung (Vergleichsbeispiel 1) versus Kreislauffahrweise (Beispiel 2) gemäß der vorliegenden Erfindung kann festgestellt werden, dass der Mehrbedarf an Strom und Kondensat durch den Minderverbrauch an Stickstoff, aber vor allen Dingen durch die höhere Verfügbarkeit der Anlage, die sich durch eine höhere Produktionsleistung bemerkbar macht, mehr als kompensiert wird. Der Dampfverbrauch hält sich in etwa die Waage. Die für die Reinigungsmaßnahme gesparte Zeit beträgt 3 Stunden und 48 Minuten, was einer verbesserten Produktionsleistung von 190 Tonnen Nitrobenzol entspricht.

**Vergleichsbeispiel 2:** Kurzstillstand der Produktionsanlage mit Komplettabstellung der Anlage, Reparaturmaßnahme und Wiederanstellung der Anlage.

Kurzstillstand der Anlage für eine Reparaturmaßnahme in der Wäsche: Dazu wurde die Anlage komplett abgefahren, also Nitrierung, Wäschen und Destillation. Die Energien waren während der Reparaturmaßnahme abgestellt. Nach der Reparatur wurde wieder angefahren, wobei die komplette Anlage inertisiert, befüllt und aufgeheizt werden musste.

### Durchführung der Komplettabstellung der Anlage:

Die Anlage wurde, wie in Beispiel 1 beschrieben, abgestellt. Die Komplettabstellung dauerte ohne Spülung und Entleerung der Apparate, Pumpen und Rohrleitungen wieder zwei Stunden, wenn man vom Abfahren (Kaltfahren) der TDZ absieht.

### Durchführung der Reparaturmaßnahme:

Undichte Rohrleitung in der neutralen Wäsche abdichten: Dafür wurde die betroffene Rohrleitung in den nachgeschalteten Apparat der Wäsche mit 10 m₃ Stickstoff leergeblasen. Dann wurde mit 2 m₃ Kondensat gespült und die Rohrleitung entleert. Anschließend wurde die defekte Dichtung der Rohrleitung ersetzt. Die Reparaturmaßnahme dauerte insgesamt 1,5 Stunden. In modernen, automatisierten Produktionsanlagen spielt die Personalstärke für die Vorbereitung der Reparatur, nämlich das Spülen der Rohrleitung, eine wichtige Rolle. In diesem Fall wird ein zusätzlicher Produktionsmitarbeiter benötigt. Handwerker zur Demontage und Montage der Rohrleitung, um die defekte Dichtung zu ersetzten, werden ebenfalls benötigt.

### Durchführung der Wiederanstellung der Anlage:

Vorab wurden in der gesamten Produktionsanlage die Vakuumpumpen in Betrieb genommen. Anschließend wurde die Anlage, wie in Verglcichsbcispicl 1 beschrieben, wieder angestellt. Die Anlage war nach 4,5 Stunden wieder angefahren und konnte auf Nennlast hochgezogen werden.

### Bilanz des Energie- und Zeitbedarfes für Ab- und Anfahren der Anlage inklusive der Reinigungsmaßnahme:

Der gesamte Zeitbedarf für die Maßnahme betrug 8,5 Stunden, da genügend Personal vorhanden war und keine technischen Schwierigkeiten auftraten. Der Zeitbedarf für die Reparatur an sich betrug 1,5 Stunden. Für das Abfahren wurden 2,5 Stunden benötigt. Das Anfahren dauerte 4,5 Stunden. Insgesamt gingen so 450 Tonnen an Produktion von Nitrobenzol verloren. Der Dampfverbrauch betrug 3,4 Tonnen 6 bar Dampf und 8 Tonnen 16 bar Dampf und 4,8 Tonnen 110 bar Dampf. Beim Abfahren der Anlage wurde kein Dampf verbraucht. Es wurden insgesamt 610 Nm³ Stickstoff benötigt, davon 550 Nm³ für das Abfahren und 50 Nm³ für das Wiederanfahren der Anlage, weitere 10 Nm³ für die Reparaturmaßnahme.

Der Verbrauch an Kondensat betrug 15,5 m³ (2 m³ für das Spülen der Rohrleitung und 13,5 m³ für das Anfahren der neutralen Wäsche). Der Verbrauch an Strom betrug insgesamt 5470 kW. Für das Abfahren der Anlage wurden 440 kW für die TDZ, 180 kW für die Nitrierung und 445 kW für die Wäschen benötigt. Für das Anfahren der Anlage wurden 4005 kW für die Wäschen und 400 kW für die Kreislaufschwefelsäurepumpen benötigt. Während der Reparaturmaßnahme wurde kein Strom verbraucht.

**Beispiel 2:** Kurzstillstand der Produktionsanlage mit Kreislauffahrweise der nicht von der Reparaturmaßnahme betroffenen Anlagenteile, Reparaturmaßnahme und Wiederanstellung der Anlage.

Kurzstillstand der Anlage für Reparaturarbeiten in der Nitrobenzolwäsche: Dazu wurden die Wäschen und die Destillation komplett abgefahren. Die anderen Anlagenteile wie die Nitrierung, die saure und die alkalische Abwasseraufarbeitung wurden in Kreislauffahrweise gestellt. Die Energien während der Reparaturarbeiten waren nur in der Wäsche und der Destillation abgestellt (Vakuum blieb in stand-by). Nach der Reparatur wurde wieder angefahren.

### Durchführung der Komplettabstellung der Wäschen und Destillation und Einstellung der restlichen Anlagenteile auf Kreislauffahrweise:

Als erstes wurde die Sauerwasseraufarbeitung in Kreislauffahrweise gestellt, indem die Ausschleusung des sauren Abwassers in den Abwasserkanal mittels der Sumpfpumpe des Strippers, die 10 KW/h benötigt, zurück in den Sauerwasservorlagetank gestellt wurde. Der Dampf zum Sauerwasserstripper wurde von 1,2 t/h auf 0,7 t/h 6 bar Dampf gedrosselt und die Einspeisung von Sauerwasser aus dem Sauerwasservorlagetank mittels der Sauerwasserpumpe, die 10 KW/h benötigt, in den Sauerwasserstripper wurde von 20 m³ auf 13 m³ reduziert. Das Umstellen der Sauerwasseraufarbeitung auf Kreislauffahrweise erfolgte mittels Automatik in 29 Sekunden.

Als nächstes wurde die alkalische Abwasseraufarbeitung in Kreislauffahrweise gestellt, indem die Ausschleusung des alkalischen Abwassers der thermische Druckzersetzung (TDZ) in den Abwasserkanal mittels der Hochdruckpumpe der TDZ, die 55 KW/h benötigt, zurück in die alkalische Abwasservorlage gestellt wurde. Der Dampf zur Druckzersetzung wurde von 0,32 t/h auf 0,20 t/h 110 bar Dampf gedrosselt und die Einspeisung von alkalischem Abwasser aus der alkalischen Abwasservorlage in die TDZ wurde von 4,0 m³/h auf 2,5 m³/h reduziert. Das Umstellen der TDZ auf Kreislauffahrweise erfolgte mittels Automatik in 5 Minuten, weil die Reduzierung der Einspeisung zur TDZ manuell erfolgte.

Gleichzeitig wurde der Stripper des alkalischen Abwassers in Kreislauffahrweise gestellt, indem die Ausschleusung des alkalischen Abwassers in die TDZ unterbrochen wurde und mittels der Sumpfpumpe des Strippers, die 10 kW/h benötigt, das alkalische Abwasser zurück auf den Abwassertank gestellt wurde. Der Dampf zum Stripper des alkalischen Abwassers wurde von 0,4 t/h auf 0,25 t/h 6 bar Dampf gedrosselt und die Einspeisung von alkalischem Abwasser aus dem Abwassertank in den Stripper des alkalischen Abwassers wurde mittels der Einspritzpumpe des alkalischen Abwassers, die 10 kW/h benötigt, von 4 m³ auf 2,5 m³ reduziert. Das Umstellen des Strippers des alkalischen Abwassers auf Kreislauffahrweise erfolgte mittels Automatik in 31 Sekunden.

Als nächstes wurden die Einsatzstoffe zur Nitrierung abgestellt. Die Dosierpumpen der Eingangsströme von Benzol und Salpetersäure wurden abgestellt. Die Kreislaufschwefelsäure lief bei 100 °C weiter über die Nitratoren, den Phasentrennapparat, Blitzverdampfer und Kreislaufschwefelsäurevorlagetank im Kreis. Am Blitzverdampfer wurden 0,3 t/h 6 bar Dampf benötigt. Der Zeitbedarf war 1 Minute.

Als letztes wurden die Wäschen abgestellt, indem die Rohnitrobenzolzufuhr aus dem Rohnitrobenzoltank zur sauren Wäsche unterbrochen wurde. Der Rohnitrobenzolweg durch die saure, alkalische und neutrale Wäsche wurde abgestellt, indem die Förderpumpen des Rohnitrobenzols vor den jeweiligen Wäschen gestoppt wurden. Die Wäschen hatten 48 °C und blieben mit Rohnitrobenzol gefüllt. Gleichzeitig wurden der saure, der alkalische und der neutrale Waschwasserweg durch Abschalten der jeweiligen Pumpen abgestellt. Der Zeitbedarf lag bei 5 Minuten.

Dann wurde die Destillation abgestellt, indem die Einspeisung von Rohnitrobenzol unterbrochen und der Dampf zur Destillationskolonne weggenommen wurde. Unmittelbar danach wurde der Produktaustrag durch Abstellen der Sumpfpumpe unterbrochen und der Rücklauf am Kolonnenkopf durch Stoppen der Benzolpumpe abgestellt. Die Vakuumpumpe lief weiter. Der Zeitbedarf war 5 Minuten.

Die Vorbereitung (Einstellung der Kreislauffahrweise der Nitrierung und der alkalischen und sauren Abwasseraufarbeitung und das Abstellen der Wäschen und der Destillation) für die Reparaturmaßnahme dauerte ohne Spülung und Entleerung der Apparate, Pumpen und Rohrleitungen insgesamt 11 Minuten.

### Durchführung der Reparaturmaßnahme:

Die Reparaturmaßnahme wurde wie in Vergleichsbeispiel 2 beschrieben, durchgeführt. Der Zeitbedarf lag wiederum bei 1,5 Stunden. Der Dampfverbrauch während der Kreislauffahrweise betrug 1,6 Tonnen 6 bar Dampf und 0,4 Tonnen 110 bar Dampf. 400 kW Strom wurden für den Betrieb der Vakuumpumpe und Schwefelsäurekreislaufpumpe während der Kreislauffahrweise benötigt.

### Durchführung der Wiederanstellung der Anlage:

Als erstes wurden die Wäschen angestellt, indem durch Anstellen der Rohnitrobenzolpumpe die Rohnitrobenzolzufuhr aus dem Rohnitrobenzoltank zur sauren Wäsche in Betrieb genommen wurde. Danach wurden der saure, der alkalische und der neutrale Waschwasserweg durch Einschalten der jeweiligen Pumpen angestellt. Dann wurde der Rohnitrobenzolweg durch die saure, alkalische und neutrale Wäsche angestellt, indem die Förderpumpen des Rohnitrobenzols vor den jeweiligen Wäschen eingeschaltet wurden. Die Waschapparate, die mit Rohnitrobenzol und Waschwasser gefüllt waren, hatten 45 °C und erwärmten sich nach dem Anstellen der Produktionsanlage langsam wieder auf 48 °C.

Nachdem die letzte Stufe der neutralen Wäsche durch Einspeisen von 3 t/h Kondensat in Betrieb genommen war, wurde die Destillation angestellt, indem aus der letzten neutralen Wäsche 45 °C warmes Rohnitrobenzol zur Destillationskolonne gefahren wurde. Danach wurde die Sumpfpumpe der Kolonne angestellt und Rohnitrobenzol zum Rohnitrobenzoltank gefahren. Nun wurde die Destillationskolonne mit 2 t/h 16 bar Dampf beaufschlagt und auf 170 °C aufgeheizt. Bei 50 °C im Kopf der Kolonne wurde der Rücklauf durch Anstellen der Benzolpumpe in Betrieb genommen. Die Wäschen und die Destillation waren nach 1 Stunde zum Wiederanfahren der Produktionsanlage bereit.

Nun wurde die Nitrierung durch Anstellen der Benzol- und Salpctersäurepumpen mit 50 % der Nennkapazität gestartet, was einer Produktionsleistung von 25 t/h Nitrobenzol entsprach. Nach 1 Minute kam im Phasentrennapparat das Reaktionsprodukt an, der Sauerwasserstripper wurde auf Ausschleusen des sauren Abwassers und die Sumpfkolonne der Destillation auf Produktaustrag des Endproduktes Nitrobenzol gestellt. Gleichzeitig wurde der Stripper der alkalischen Abwasseraufarbeitung auf Ausschleusung zur TDZ und die TDZ von Kreislauffahrweise auf Ausschleusung in den Abwasserkanal gestellt. Das Hochziehen der Produktionsanlage auf Nennlast dauerte nochmals 1 Stunde.

### Bilanz des Energie- und Zeitbedarfes für Ab- und Anfahren aus Kreislauffahrweise der Anlage inklusive der Reparaturmaßnahme:

Der gesamte Zeitbedarf für die Maßnahme betrug 3 Stunden und 41 Minuten. Der Zeitbedarf für die Reparatur an sich betrug 1,5 Stunden. Für das Abfahren in Kreislauffahrweise wurden 11 Minuten benötigt. Das Anfahren aus der Kreislauffahrweise dauerte 1 Stunde und 1 Minute.

Insgesamt gingen so 165 Tonnen an Produktion von Nitrobenzol verloren. Der Dampfverbrauch betrugl,6 Tonnen 6 bar Dampf, 2 Tonnen 16 bar Dampf und 0,8 Tonnen 110 bar Dampf. Beim Abfahren der Anlage in Kreislauffahrweise wurde kein Dampf verbraucht. Es wurden 10 Nm³ Stickstoff für die Reparaturmaßnahme und weitere 50 Nm³ Stickstoff für das Wiederanfahren der Anlage benötigt. Der Verbrauch an Kondensat betrug 7 m³ (2 m³ für das Spülen der Rohrleitung und 5 m³ für das Ab- und Anfahren der neutralen Wäsche. Der Verbrauch an Strom betrug insgesamt 1593 kW. Für das Abfahren der Anlage wurden 178 kW für das Herunterfahren der Nitrierung in Kreislauffahrweise, 510 kW für die Kreislauffahrweise während der Reinigungsmaßnahme und 905 kW an Strom für das Anfahren der Anlage verbraucht.

### Fazit der Komplettabstellung (Vergleichsbeispiel 2) versus Kreislauffahrweise (Beispiel 2) für die Reparaturmaßnahme:

Als Fazit der Komplettabstellung (Vergleichsbeispiel 2) versus Kreislauffahrweise (Beispiel 2) gemäß der vorliegenden Erfindung kann festgestellt werden, dass bei der Kreislauffahrweise geringere Mengen an Dampf, Strom, Stickstoff und Kondensat verbraucht wurden und obendrein die Verfügbarkeit der Anlage, die sich durch eine höhere Produktionsleistung bemerkbar macht, deutlich besser war. Die für die Reparaturmaßnahme gesparte Zeit betrug 5 Stunden und 48 Minuten, was einer verbesserten Produktionsleistung von 290 Tonnen Nitrobenzol entspricht.

### Allgemeine Bedingungen für die Herstellung von MDA bei einer eingefahrenen Produktionsanlage

In einem kontinuierlichen Reaktionsprozess (Schritt a)) wurden 24,3 t/h Einsatzanilin (enthaltend 90 Massen-% Anilin) und 9,9 t/h 32 %ige wässrige Formaldehydlösung (Formalin) (molares Verhältnis Anilin zu Formaldehyd 2,1 zu 1) vermischt und bei 90 °C und 1,4 bar absolut in einem gerührten Reaktionskessel zum Aminal umgesetzt. Der Reaktionskessel war mit einem Kühler mit Kühlkreislaufpumpe versehen. Das den Reaktionskessel verlassende Reaktionsgemisch wurde in einen Phasentrennapparat (Aminalabscheider) geführt (Schritt b)). Nach der Phasentrennung zur Entfernung der wässrigen Phase wurde die organische Phase in einer Mischdüse mit 30 %iger wässriger Salzsäure versetzt (Protonierungsgrad 10 %, d. h., pro Mol Aminogruppen werden 0,1 Mol HCl zugegeben) und in den ersten Umlagerungsreaktor gefahren. Die Umlagerungsreaktion wurde in einer Reaktorkaskade bei 45 °C bis 165 °C durchgeführt (Schritt c)). Nach vollständiger Reaktion wurde das erhaltene Reaktionsgemisch mit 32 %iger Natronlauge im molaren Verhältnis von 1,1:1 Natronlauge zu HCl versetzt und in einem Neutralisationsrührbehälter umgesetzt (Schritt d)). Die Temperatur betrug dabei 115 °C und der absolute Druck 1,4 bar. Die neutralisierte Reaktionsmischung wurde anschließend in einem Neutralisationsabscheider in eine wässrige, untere Phase, die zu einem Abwassersammelbehälter geführt wurde, und in eine organische Phase getrennt (Schritt e)). Die organische, obere Phase wurde zur Wäsche geleitet und in einem gerührten Waschbehälter mit Kondensat und/oder Wasser aus dem Seitenstrom der Abwasserkolonne (Anilin/Wassergemisch) gewaschen (Schritt f)). Nach Abtrennung des Waschwassers in einem Waschwasserabscheider (Schritt g)) wurde das so erhaltene Roh-MDA durch Destillation von Wasser und Anilin befreit, wobei als Sumpfprodukt 17 t/h MDA anfielen (Schritt h)).

### Vergleichsbeispiel 3: Herunterfahren der Anlage bis zum Komplettstillstand für eine Reparatur und wieder anfahren der Anlage

Als erstes wurde die gesamte Produktionsanlage aus Beispiel 1 auf eine Produktionslast von 10 t/h MDA gebracht, um die Anlage möglichst zügig mit Anilin freispülen zu können, aber auch möglichst wenig Abfallprodukt, wie Anilin, Roh-MDA und Abwasser, die alle wieder aufbereitet werden müssen, anfallen zu lassen.

Die Abstellung der Anlage wurde mit dem Abstellen des Eingangsstromes von Formaldehyd in den Aminalreaktor begonnen. Dazu wurde die Formaldehydpumpe gestoppt und der Formaldehydweg vom Formaldehydvorratstank mit Wasser für 10 Minuten von Formaldehyd frei gespült. Nun wurde der Aminalteil der Anlage 3 Stunden mit Anilinmenge verdünnt, wobei restliches Formaldehyd zu Aminal abreagierte und aus dem Aminalreaktor ausgespült wurde. Während des Spülvorgang wurde die Anilinmenge so erhöht, dass ein Ausgleich für die jetzt fehlende Menge an Aminal stattfand, um einen gleichbleibenden Massenstrom zu gewährleisten, und die Stände in den Folgeapparaten nicht reduzieren zu müssen. Die Reaktionswärme fiel nach Stoppen der Formalinzufuhr nicht mehr an und der Aminalreaktor kühlte sich auf 67 °C ab. Nach 3 Stunden wurde die Anilinzufuhr gestoppt, der Kühlkreislauf abgestellt und der Aminalkühler, die Aminalpumpe und der Aminalrührbehälter nacheinander in den Aminalabscheider restentleert. Der Druck im Aminalkessel verblieb während des Spülvorganges bei 1,4 bar absolut. Der Aminalabscheider wurde nun ebenfalls restentleert, indem das Spülanilin und das über dem Anilin stehende Restwasser in den ersten Umlagerungsreaktor gefahren wurden. Nun stand der Aminalteil. Das Abfahren hat insgesamt 5 Stunden gedauert.

Als nächstes wurde die Reaktorkaskade der Umlagerungsreaktion abgefahren. Hier wurde bereits 2 Stunden nach Beginn der Abstellung des Aminalteiles der Anlage die Reaktorkaskade mit mehr Dampf beaufschlagt, um die wegbrechende Reaktionswärme zu kompensieren. Die Temperaturen in der Reaktorkaskade wurden bei 45 °C bis 165 °C belassen. Das Abfahren der Reaktorkaskade wurde mit der Beendigung der Eindosierung von der 30 %igen wässrigen Salzsäure in die Mischdüse vor dem ersten Umlagerungsreaktor zu dem Zeitpunkt, als die Restentleerung des Aminalabscheiders gestartet wurde, begonnen. Dann wurden die Reaktoren der Reaktorkaskade nacheinander zur Neutralisation leergefahren. Dampf und Vakuum wurden abgestellt, als der letzte Umlagerungstank leer war. Nun stand die Reaktorkaskade der Umlagerungsreaktion. Das Abfahren hat insgesamt 3 Stunden gedauert.

Danach wurde die Neutralisation außer Betrieb genommen, indem 10 Minuten länger als verdünntes Reaktionsgemisch aus der Reaktorkaskade der Umlagerungsreaktion 32 %ige Natronlauge in den Neutralisationsrührbehälter nachgefahren wurde. Dann wurde der Inhalt des Neutralisationsrührbehälters und -abscheiders in einen alkalischen Ablassbehälter restentleert. Der absolute Druck blieb bei 1,4 bar. Nun stand die Neutralisation. Der Abfahrvorgang mit Restentleerung hatte 2 Stunden gedauert.

Als nächstes wurde die Wäsche außer Betrieb genommen, indem zuerst Kondensat und/oder Wasser aus dem Seitenstrom der Abwasserkolonne (Anilin/Wassergemisch) zum gerührten Waschbehälter geschlossen wurde. Der Rührer des Waschbehälters wurde abgestellt und der Inhalt des Waschbehälters in den Waschwasserabscheider entleert. Der Inhalt des Waschwasserabscheiders wurde in die Destillationsvorlage entleert. Nun stand die Wäsche. Der Abfahrvorgang hatte 2 Stunden gedauert.

Als letztes wurde die Destillation abgestellt, indem nach der Restentleerung der Wäsche die komplette Destillation auf Kreislauffahrweise gestellt wurde, wobei das in der Destillation vorhandene Roh-MDA mit 6 t/h Anilin aus dem Anilinvorratstank verdünnt wurde. Der Dampf zur Destillation wurde abgestellt. Die Destillation wurde innerhalb von 4 Stunden über das noch anstehende Vakuum kalt gefahren. Im Anschluss wurde das Vakuum abgestellt und der Inhalt der kompletten Destillation (Destillationsvorlage, Wärmetauscher, Vordestillationskolonne mit Kondensationssystem, MDA-Kolonne mit Sumpfentnahme, Dampferzeuger) in den alkalischen Ablassbehälter entleert. Nun stand die Destillation, wobei der Abfahrvorgang 4 Stunden gedauert hat.

Während des Kaltfahrens der Destillation wurde die Abwasseraufarbeitung außer Betrieb genommen, indem zuerst das Anilin/Wassergemisch aus der Abwasserextraktion, die aus Abwassersammelbehälter, Abwassererhitzer und Anilintrennbehälter bestand, in den Abwassertank entleert wurde. Die Abwasserdestillation, bestehend aus einem Wärmetauscher, einer Prozessabwasserdestillationskolonne mit Kondensationssystem, einem Prozessabwasserkühler und einer Seitenstromvorlage der Prozessabwasserdestillationskolonne, wurde abgestellt, indem der Dampf zur Abwasserdestillation abgestellt und im Anschluss der Inhalt der Abwasserdestillation in den Abwassertank entleert wurde.

Jetzt stand die komplette MDA-Anlage, die zu diesem Zeitpunkt auch komplett entleert war. Der Anlagendruck wurde auf Umgebungsdruck gestellt, indem alle Druckhaltungen der Anlage außer Betrieb genommen wurden. Die Restentleerungen aller Anlagenteile wurden nochmals geöffnet, um Reststoffe aus der Anlage abzulassen. Die Komplettabstellung mit Entleerung sämtlicher Apparate, Pumpen und Rohrleitungen dauerte insgesamt 24 Stunden.

**Verbrauch:** Es wurden 20 Nm³ Stickstoff zum Vakuum brechen und 500 kW Strom zur Kreislauffahrweise der Destillation verbraucht. Des Weiteren fiel ein erhöhter Dampfbedarf in der Reaktorkaskade der Umlagerungsreaktion von 5 Tonnen 6 bar Dampf und 5 Tonnen 16 bar Dampf an. Außerdem gab es einen Anfall von 10 Tonnen Spülanilin, das vor dem Einsatz in der Aminalreaktion aufbereitet werden musste.

### Durchführung einer eintägigen Instandhaltungsmaßnahme

In dem Waschbehälter musste ein defektes Rührorgan ersetzt werden.

### Vorbereitung zur Wiederinbetriebnahme der Anlage

Dabei wurden alle Anlagenteile zuerst in Kreislauffahrweise gebracht. Das Wiederanstellen der Anlage begann mit der parallelen Inbetriebnahme aller Kreisläufe der Gesamtanlage. Zuerst wurden die Anlagenteile mit Anilin und/oder Hilfsstoffen wie HCl oder NaOH befüllt.

### Befüllung des Aminalteiles und Einstellung der Kreislauffahrweise:

Zuerst wurde die Anilinvorlage mit Frischanilin aus dem Anilinvorratstank befüllt. Dann wurde der leere Aminalreaktor mit Anilin befüllt, bis Anilin über das Siphon in den Aminalabscheider überlief. Als der Aminalabscheider zur Hälfte mit Anilin befüllt war, wurde der Anilinstrom zum Aminalreaktor abgestellt und die Aminalkreislauffahrweise mittels der Pumpe vom Aminalabscheider in Gang gebracht. 4 t/h Anilin wurden nun im Kreis vom Aminalabscheider über den Aminalreaktor gepumpt. Zeitbedarf: 3 Stunden.

### Befüllung der Reaktorkaskade der Umlagerungsreaktion und Einstellung der Kreislauffahrweise:

Der erste Umlagerungsreaktor wurde bis zu einem Stand von 60 % mit Frischanilin aus dem Anilinvorratstank befüllt. Dann wurde der Anilinstrom abgestellt und der erste Umlagerungsreaktor mit 24 t/h Frischanilin mittels der Austragspumpe im Kreis gepumpt. Die restlichen Umlagerungsreaktoren der Reaktorkaskade wurden aus dem sauren Ablassbehälter mit einer Mischung, die aus Anilin, Salzsäure und Spuren an Roh-MDA bestand, befüllt und die Umlagerungskreislauffahrweise mittels der Pumpen der Umlagerungsreaktoren vom letzten Umlagerungsreaktor zum zweiten Umlagerungsreaktor in Gang gebracht. 10 t/h der Mischung aus dem sauren Ablassbehälter wurden nun im Kreis gepumpt und mit Dampf auf 100 °C aufgeheizt. Die restlichen 15 Tonnen der Mischung aus dem sauren Ablassbehälter mussten später bei laufender Produktion beigemischt werden, was allerdings eine Schwankung im 2-Kerngehalt des Endproduktes bedeutet. Zeitbedarf: 8 Stunden.

### Befüllung der Neutralisation und Einstellung der Kreislauffahrweise:

2 Tonnen 32 %ige Natronlauge aus dem Natronlaugevorratstank und 8 Tonnen Kondensat aus dem Kondensatvorratsbehälter wurden in den Neutralisationsrührbehälter gefahren. Der Neutralisationsrührbehälter war nun gefüllt und 2 Tonnen der verdünnten Natronlauge über das Syphon in den Neutralisationsabscheider abgelaufen. Mittels der Pumpe des Neutralisationsabscheiders wurde die Kreislauffahrweise in Gang gebracht, indem die verdünnte Natronlauge aus dem Neutralisationsabscheider in den Neutralisationsrührbehälter gepumpt wurde. 4 t/h verdünnte Natronlauge wurde nun im Kreis vom Neutralisationsabscheider über den Neutralisationsrührbehälter gepumpt. Zeitbedarf: 4 Stunden.

### Die Wäsche wurde nicht befüllt und auch nicht in Kreislauffahrweise gestellt:

Der Rührbehälter der Wäsche und der daran angeschlossene Abscheider blieben bis zum Anfahren der Anlage leer.

### Befüllung der Destillation und Einstellung der Kreislauffahrweise:

Die Destillationsvorlage wurde mit Frischanilin aus dem Anilinvorratstank bis auf 60 % Füllstand befüllt. Aus der Destillationsvorlage wurde dann die komplette Destillation bestehend aus Wärmetauscher, Vordestillationskolonne mit Kondensationssystem, MDA-Kolonne mit Sumpfentnahme und Dampferzeuger mit Frischanilin befüllt, Frischanilin abgestellt und 10 t/h Frischanilin über die Vordestillationskolonne und MDA-Kolonne im Kreis gefahren. Anschließend wurde das Vakuum der Destillation in Betrieb genommen und die gesamte Destillation mit Dampf auf 100 °C erhitzt. Zeitbedarf: 5 Stunden.

### Befüllung der Abwasseraufarbeitung und Einstellung der Kreislauffahrweise:

Aus dem Abwassertank wurde Abwasser in den Abwassersammelbehälter gepumpt. Aus dem Abwassersammelbehälter wurde nun Abwasser in den Abwassererhitzer und Anilintrennbehälter gefördert. Frischanilin aus dem Anilinvorratstank wurde nun auf den Abwassererhitzer gegeben, der Abwassererhitzer anschließend auf 90 °C aufgeheizt und das Gemisch aus Frischanilin und Abwasser aus dem Abwassererhitzer über den Anilintrennbehälter und den Abwassersammelbehälter im Kreis gefahren. Die Abwasserdestillation blieb bis zum Anfahren der Anlage außer Betrieb. Zeitbedarf: 5 Stunden.

Insgesamt werden 15 Stunden benötigt, um die Gesamtanlage, wie beschrieben, in Kreislauffahrweise zu versetzen, weil Teile der Anlage parallel befüllt wurden. Dazu wurden 50 Tonnen 16 bar an Dampf und 9500 kW an Strom zum Betreiben der Motoren benötigt.

### Wiederinbetriebnahme der Anlage

Die Anlage lief wie in den Vorbereitungen zur Wiederinbetriebnahme der Anlage beschrieben in den einzelnen Betriebssegmenten im Kreis, d.h. sie war aufgeheizt, Rührer waren in Betrieb, in den benötigten Bereichen lag Beschleierungsdruck mit Stickstoff und Vakuum an. Einsatzstoffe und Hilfsstoffe standen bereit.

### Anfahren der Destillation mit Vakuumsystem:

Die Destillation mit Vakuumsystem stand in Kreislauffahrweise. Das Vakuumsystem der Vordestillationskolonne und MDA-Kolonne wurde in Betrieb genommen und auf 120 mbar absolut eingestellt. Dann wurde der 16 bar Dampf (Verbrauch: 40 Tonnen) zur Vordestillationskolonne und der 110 bar Dampf (Verbrauch: 10 Tonnen) zur MDA-Kolonne geöffnet und die Kolonnen aufgeheizt. Die Temperatur in der Vordestillationskolonne betrug 190 °C und in der MDA-Kolonne 225 °C. Das zum Destillieren notwendige Anilin wurde für die Zeit der Kreislauffahrweise aus der Anilinvorlage in die Pumpenvorlage der Destillation eingespeist. Der Dampferzeuger war in Betrieb. Nun war das Betriebssegment der Destillation bereit, Roh-MDA aufzunehmen. Zeitbedarf: 3 Stunden.

### Anfahren der Aminalreaktion:

30 Minuten bevor die Destillation bereit war, Roh-MDA aufzunehmen, wurde die Aminalherstellung gestartet, indem Anilin zum Aminalreaktor geöffnet und 10 Minuten später der Formalinstrom angestellt wurde. Gleichzeitig wurde der Weg der 90 °C heißen organischen Phase aus dem Aminalabscheider zum ersten Reaktor der Umlagerungsreaktion geöffnet und die Temperatur im ersten Umlagerungsreaktor wurde mittels Vakuum auf 50 °C abgesenkt. Jetzt konnte die saure Katalyse der Umlagerungsreaktion mit Salzsäure gestartet werden. Das im Aminalabscheider anfallende Aminalwasser wurde der Abwasseraufarbeitung zugeführt. Nun war das Betriebssegment der Aminalreaktion angestellt und Aminallösung ging zur Umlagerungsreaktion. Zeitbedarf: 15 Minuten.

### Anfahren der Umlagerungsreaktion:

Nachdem der Salzsäurestrom in Betrieb genommen wurde und die Temperatur im ersten Umlagerungsreaktor stand, wurden die weiteren Umlagerungsreaktoren und Verweilzeittürme der Reaktorkaskade auf 60 °C bis zum letzten Reaktor auf 165 °C aufgeheizt (Verbrauch: 60 Tonnen 16 bar Dampf). Nun war das Betriebssegment der Umlagerungsreaktion angestellt und die Kondensationslösung, bestehend aus MDA, Anilin und Salzsäure (Roh-MDA) wurde als nächstes neutralisiert. Zeitbedarf: 10 Minuten.

### Anfahren der Neutralisation:

Die Natronlaugedosiereinrichtung wurde in Betrieb genommen, indem Natronlauge und Waschwasser zum Neutralisationsrührbehälter gefahren wurden. 10 Minuten später wurde der Weg der sauren Kondensationslösung aus der Umlagerungsreaktion aufgeschaltet. Nun war das Betriebssegment der Neutralisation angestellt und Roh-MDA konnte gewaschen werden. Zeitbedarf: 10 Minuten.

### Anfahren der Wäsche:

116 °C heißes neutralisiertes Roh-MDA kam im MDA-Wäscher an und wurde mit Kondensat gewaschen. Die Waschwasserzugabe, bestehend aus Kondensat und/oder dem Seitenstrom der Prozessabwasserkolonne, wurde angestellt. Nun war das Betriebssegment der Wäsche angestellt. Roh-MDA verließ den Phasentrennapparat und ging zur Destillation. Zeitbedarf: 5 Minuten.

### Anfahren der Abwasseraufarbeitung:

Sobald die Neutralisation und Wäsche liefen, wurde die Abwasseraufarbeitung angestellt, indem die Abwasserextraktion und die Abwasserdestillation in Betrieb genommen wurden. Dazu wurde das anfallende Abwasser aus den vorbeschriebenen Verfahrensschritten (Neutralisation, Wäsche und Destillation), das im Abwassersammelbehälter ankam, mittels Pumpe über den Prozessabwassererhitzer in den Anilintrennbehälter gefahren. Von dort aus ging das extrahierte Abwasser in die Abwasserdestillation. Die Abwasserdestillation wurde mit 20 Tonnen 6 bar Dampf auf 107 °C erhitzt, und das Abwasser verließ die Produktionsanlage. Zeitbedarf: 2 Stunden.

Die komplette MDA-Anlage lief jetzt mit einer reduzierten Last von 10 t/h MDA und konnte nun auf die gewünschte Sollproduktion hochgefahren werden. Insgesamt wurden 10 Stunden benötigt, um die Gesamtanlage, wie beschrieben, aus der Kreislauffahrweise in Betrieb zu nehmen und erstes Endprodukt in den MDA-Tank auszuschleusen. Dazu wurden 100 Tonnen 16 bar Dampf, 10 Tonnen 110 bar Dampf und 20 Tonnen 6 bar Dampf sowie 6315 kW Strom zum Betreiben der Motoren benötigt.

Es war dabei zwingend erforderlich, die Produktionsanlage mit reduzierter Last anzufahren, da ansonsten die benötigten Temperaturprofile für Aminal- und Umlagerungsreaktion, Neutralisation, Wäsche und Abwasseraufarbeitung und Destillation nicht schnell genug zur Verfügung stehen. Dieses würde zu unvollständigen Reaktionen, vermehrten Nebenprodukten und mangelhafter Aufarbeitung des Produktes führen.

### Fazit:

Der Zeitaufwand für den gesamten Produktionsstillstand (Abfahren, Maßnahme und Anfahren) umfasste 73 Stunden.

Der Energieverbrauch dafür (Abfahren, Maßnahme und Anfahren) betrug 15815 kW Strom, 105 Tonnen 16 bar Dampf, 10 Tonnen 110 bar Dampf und 25 Tonnen 6 bar Dampf. Darüber hinaus wurden Hilfsstoffe in Form von 20 Nm³ Stickstoff zum Brechen des Vakuums verbraucht.

**Beispiel 3:** Anlage in Kreislauffahrweise bringen, Reparatur in der Wäsche, Anlage aus der Kreislauffahrweise wieder anfahren.

Als erstes wurde die gesamte Produktionsanlage wie im Vergleichsbeispiel 3 auf die optimale Produktionslast von 10 t/h MDA gebracht, um danach die gesamte Anlage in Kreislauffahrweise zu bringen.

Die eigentliche Einstellung der Anlage auf Kreislauffahrweise begann mit dem Abstellen des Eingangsstromes von Formalin in den Aminalreaktor. Dazu wurde die Formaldehydpumpe gestoppt und der Formaldehydweg vom Formaldehydvorratstank mit Wasser für 10 Minuten von Formaldehyd frei gespült. Nun wurde der Aminalteil der Anlage 30 Minuten mit Anilin verdünnt, wobei Formaldehyd weiterhin zu Aminal abreagierte und die Aminallösung verdünnt wurde. Während des Spülvorganges wurde die Anilinmenge so erhöht, dass ein Ausgleich für die jetzt fehlende Menge an Aminal stattfand, um einen gleichbleibenden Massenstrom zu gewährleisten und um die Stände in den Folgeapparaten nicht reduzieren zu müssen. Die Reaktionswärme fiel nach Stoppen der Formaldehydzufuhr nicht mehr an und der Aminalreaktor kühlte sich auf 67 °C ab. Nach 30 Minuten wurde die Anilinzufuhr gestoppt und der Aminalteil der Anlage auf Kreislauffahrweise gestellt, indem das mit Anilin verdünnte Aminal ungekühlt vom Aminalreaktor über das Siphon in den Aminalabscheider und von dort im Kreis zurück zum Aminalreaktor gepumpt wurde. Der Druck im Aminalkessel verblieb während der Kreislauffahrweise bei 1,4 bar absolut. Das Einstellen des Aminalteiles der Anlage in Kreislauffahrweise dauerte insgesamt 1 Stunde.

Als nächstes wurde die Reaktorkaskade der Umlagerungsreaktion in Kreislauffahrweise gebracht, indem zunächst der Salzsäurestrom und dann der Aminalstrom abgestellt wurden. Die Kondensationslösung, bestehend aus MDA, Anilin und Salzsäure konnte dann ohne Beheizung vom letzten Umlagerungsreaktor in den ersten Umlagerungsreaktor und über die Reaktorkaskade im Kreis gepumpt werden. Das Einstellen des Umlagerungsteiles der Anlage auf Kreislauffahrweise dauerte insgesamt 1 Stunde.

Als nächstes wurde die Neutralisation in Kreislauffahrweise gestellt, indem zuerst die saure Kondensationslösung, die aus der Umlagerungsreaktion kam, und 10 Minuten später die 32 %ige Natronlauge und das Waschwasser abgestellt wurden. Dann wurde der Inhalt des Neutralisationsabscheiders mittels der Kreislaufpumpe aus dem Neutralisationsabscheider in den Neutralisationsrührbehälter über den Siphon zurück in den Neutralisationsabscheider gepumpt. Damit stand die Kreislauffahrweise. Der absolute Druck in der Neutralisation blieb bei 1,4 bar absolut. Das Einstellen des Neutralisationsteiles der Anlage auf Kreislauffahrweise dauerte insgesamt 40 Minuten.

Als nächstes wurde die Wäsche abgestellt, indem die Waschwasserzugabe, bestehend aus Kondensat, zum gerührten Waschbehälter geschlossen wurde. Der Rührer des Waschbehälters wurde abgestellt. Um die Reparaturmaßnahme in der Wäsche vorzubereiten, wurde der Inhalt des Waschbehälters in den Waschwasserabscheider entleert. Der Inhalt des Waschwasserabscheiders wurde in die Destillationsvorlage entleert. Nun stand die Wäsche. Der Abfahrvorgang dauerte 2 Stunden.

Als letztes wurde die Destillation auf Kreislauffahrweise gestellt, indem nach der Restentleerung der Wäsche das in der Destillation vorhandene Roh-MDA mit 6 t/h Anilin aus dem Anilinvorratstank verdünnt wurde. Es kam kein Roh-MDA in der Pumpenvorlage der Destillation mehr an. Der Sumpfablauf der MDA-Kolonne wurde über den Dampferzeuger und den Wärmetauscher zurück auf die Pumpenvorlage der Destillation gestellt und somit über die Pumpenvorlage, den Wärmetauscher, die Vordestillationskolonne und zurück in den Sumpf der MDA-Kolonne im Kreis gefahren. Nun konnte der Dampf zur Vordestillationskolonne und MDA-Kolonne abgestellt werden. Anschließend konnte das Vakuumsystem der beiden Kolonnen abgestellt werden. Das Einstellen des Destillationsteiles der Anlage auf Kreislauffahrweise dauerte insgesamt 3 Stunden.

Zuletzt wurde die Abwasseraufarbeitung auf Kreislauffahrweise genommen, als kein Prozesswasser mehr anfiel. Die Abwasserextraktion, bestehend aus Abwassersammelbehälter, Abwassererhitzer und Anilintrennbehälter, wurde auf Kreislauffahrweise gestellt, indem der Ablauf des Anilintrennbehälters auf den Abwassersammelbehälter gestellt und mittels Pumpe über den Prozessabwassererhitzer in den Anilintrennbehälter im Kreis gepumpt wurde. Die Kreislauffahrweise konnte ohne Beheizung auf unbestimmte Zeit betrieben werden. Die Abwasserdestillation, bestehend aus einem Wärmetauscher, einer Prozessabwasserdestillationskolonne mit Kondensationssystem, einem Prozessabwasserkühler und einer Seitenstromvorlage der Prozessabwasserdestillationskolonne, wurde abgestellt, indem der Dampf zur Kolonne abgestellt wurde. Eine Kreislauffahrweise der Abwasserdestillation war hierbei nicht vorgesehen.

Jetzt lief die komplette MDA-Anlage bis auf die Wäsche in Kreislauffahrweise. Das Einstellen der Kreislauffahrweise hatte 6 Stunden in Anspruch genommen.

**Verbrauch:** 20 Nm³ Stickstoff zum Vakuum brechen, und 3825 kW Strom um die Anlage in Kreislauffahrweise zu bringen, Anfall von 10 Tonnen Spülanilin in der Destillation, das vor dem Einsatz in der Aminalreaktion aufbereitet werden musste.

### Durchführung einer eintägigen Instandhaltungsmaßnahme

An dem Waschbehälter musste ein defektes Schauglas und eine leckende Dichtung gewechselt werden. Für die Kreislauffahrweise während der Maßnahme wurden 15300 kW an Strom benötigt. Es wurde lediglich ein wenig Dampf verbraucht, um die Kreisläufe auf Temperatur zu halten (12 Tonnen 16 bar Dampf).

### Vorbereitung zur Wiederinbetriebnahme der Anlage

Die Vorbereitungen zur Wiederinbetriebnahme der Anlage entfielen, da schon alle Anlagenteile in Kreislauffahrweise liefen. Daher entfiel auch das Befüllen der Anlagenteile mit Anilin und/oder Hilfsstoffen wie Salzsäure oder Natronlauge.

### Wiederinbetriebnahme der Anlage

Die Anlage lief, wie zuvor in den Vorbereitungen zur Wiederinbetriebnahme der Anlage beschrieben, in den einzelnen Betriebssegmenten im Kreis. Einsatzstoffe und Hilfsstoffe standen bereit, die Anlagenteile waren aufgeheizt, Rührer waren in Betrieb, in den benötigten Bereichen lag Beschleierungsdruck mit Stickstoff an, und Vakuum lag ebenso an.

Die Wiederinbetriebnahme der Anlage wurde, wie im Vergleichsbeispiel 3 beschrieben, durchgeführt. Die komplette MDA-Anlage lief jetzt mit einer reduzierten Last von 10 t/h MDA und konnte nun auf die gewünschte Sollproduktion hochgefahren werden. Insgesamt wurde wieder 10 Stunden benötigt, um die Gesamtanlage, wie beschrieben, aus der Kreislauffahrweise in Betrieb zu nehmen und erstes Endprodukt in den MDA-Tank auszuschleusen. Dazu wurden ebenfalls 100 Tonnen 16 bar Dampf, 10 Tonnen 110 bar Dampf und 20 Tonnen 6 bar Dampf sowie 6315 kW Strom zum Betreiben der Motoren benötigt.

Der Zeitaufwand für die gesamte Aktion (Abfahren, Ausführen der Maßnahme und Anfahren) betrug 40 Stunden. Somit ergab sich bei einer Nennlast von 380 Tagestonnen eine Mehrproduktion von 522,5 Tonnen MDA im Vergleich zu Vergleichsbeispiel 3.

Der Energieverbrauch für die gesamte Aktion (Abfahren, Maßnahme und Anfahren) umfasste 25500 kW Strom, 112 Tonnen 16 bar Dampf, 10 Tonnen 110 bar Dampf und 25 Tonnen 6 bar Dampf sowie einen Verbrauch von Hilfsstoffen in Form von 20 Nm³ Stickstoff zum Vakuum brechen.

Fazit: Es werden im erfindungsgemäßen Beispiel 3 mit Kreislauffahrweise 7 Tonnen 16 bar Dampf und 9685 kW Strom mehr verbraucht als bei einer Komplettabstellung der Anlage wie in Vergleichsbeispiel 3. Dafür ergibt sich aber eine stark verbesserte Produktivität der Anlage, da wegen des geringeren Zeitbedarfes für die ganze Aktion (Abfahren, Maßnahme und Anfahren) über 500 Tonnen an MDA mehr produziert werden konnten.

## Patentansprüche

1. Verfahren zur Herstellung eines chemischen Produktes oder einer chemischen Zusammensetzung, wobei das Verfahren die nachfolgenden Schritte umfasst und in einer Anlage aufweisend diesen Schritten entsprechende Anlagenteile durchgeführt wird:
(I) Reaktion von mindestens einem Substrat in einem Reaktor unter Bildung mindestens eines chemischen Produktes oder einer chemischen Zusammensetzung, wobei das mindestens eine Substrat mit einem Massenstrom m₁ in den Reaktor eingetragen wird;
(II) Aufarbeitung des in Schritt (I) erhaltenen Reaktionsgemisches in einem Aufarbeitungsapparat unter Erhalt eines Rohprodukts und mindestens eines Nebenstroms umfassend die vom Rohprodukt abgetrennten Bestandteile;
(III) Reinigung des in Schritt (II) erhaltenen Rohproduktes in mindestens einem Reinigungsapparat unter Erhalt eines gereinigten Endprodukts und mindestens eines Nebenstroms umfassend die vom gereinigten Endprodukt abgetrennten Bestandteile; und die optionalen Schritte (IV) bis (V)
(IV) Aufarbeitung des in Schritt (II) erhaltenen mindestens einen Nebenstroms in einer Aufarbeitungseinrichtung;
(V) Aufarbeitung des in Schritt (III) erhaltenen mindestens einen Nebenstroms in einer Aufarbeitungseinrichtung,
wobei
bei einem Produktionsstillstand einer Dauer von 1 Stunde bis 5 Tagen zu Revisions-, Reparatur-, Reinigungs- oder Wartungszwecken in Teilen der Anlage eines oder mehrere, jedoch nicht alle, Anlagenteile aus den Schritten (I) bis (V), insofern diese durchgeführt werden, außer Betrieb genommen werden und in diesen Anlagenteilen eine Revisions-, Reparatur-, Reinigungs- oder Wartungsmaßnahme durchgeführt wird, und wobei
der Massenstrom m₁ in Schritt (I) auf null reduziert und in jedem der nicht außer Betrieb genommenen Anlagenteile der Ausgangsstrom des Anlagenteils wieder als Eingangsstrom des jeweiligen Anlagenteiles oder eines davor liegenden Anlagenteils verwendet wird,
wobei nach der Maßnahme die gesamte Anlage unverzüglich wieder angefahren wird.

2. Verfahren gemäß Anspruch 1, umfassend die Schritte (IV) und (V).

3. Verfahren gemäß mindestens einem der vorstehenden Ansprüche, wobei mindestens zwei Substrate in dem Reaktor aus Schritt (I) zur Reaktion gebracht werden und das zweite Substrat mit einem Massenstrom m₂ in den Reaktor aus Schritt (I) eingetragen wird.

4. Verfahren gemäß mindestens einem der vorstehenden Ansprüche, weiter umfassend einen Schritt (Ia), wobei mindestens ein drittes Substrat mit dem Produkt der Reaktion aus dem Schritt (I) im Reaktor aus Schritt (I) oder in einem weiteren Reaktor zur Reaktion gebracht wird, und das mindestens eine dritte Substrat mit einem Massenstrom m₃ in den mindestens einen Reaktor aus Schritt (Ia) eingetragen wird.

5. Verfahren gemäß mindestens einem der vorstehenden Ansprüche, wobei das chemische Produkt ein Polycarbonat oder eines seiner Vorprodukte, ein Isocyanat oder eines seiner Vorprodukte, ein pharmazeutischer Wirkstoff, ein Olefin, Aromat oder Polyolefin ist.

6. Verfahren zum Betrieb einer Anlage zur Herstellung eines chemischen Produktes oder einer Produktzusammensetzung während einer Unterbrechung des Herstellungsverfahrens, die Anlage umfassend die Anlagenteile:
(I) einen Reaktor zur Ausführung der Umsetzung mindestens eines Substrats, bevorzugt mehrerer (insbesondere zweier) Substrate,
(II) einen Aufarbeitungsapparat zur Gewinnung eines Rohprodukts aus dem im Reaktor (I) erhaltenen Produktgemisch neben mindestens einem Nebenstrom enthaltend die vom Rohprodukt abgetrennten Bestandteile,
(III) einen Reinigungsapparat zur Reinigung des im Aufarbeitungsapparat (II) erhaltenen Rohprodukts zu einem gereinigten Endprodukt unter Abtrennung mindestens eines Nebenstroms enthaltend die vom gereinigten Endprodukt abgetrennten Bestandteile, sowie optional die Anlagenteile (IV) bis (V)
(IV) eine Aufarbeitungseinrichtung zur Aufarbeitung des im Aufarbeitungsapparat (II) erhaltenen mindestens einen Nebenstroms,
(V) eine Aufarbeitungseinrichtung zur Aufarbeitung des im Reinigungsapparat (III) erhaltenen mindestens einen Nebenstroms,
das Verfahren umfassend die Schritte
(i) Stoppen der Zufuhr des mindestens einen Substrats, bevorzugt aller Substrate in den Reaktor (I),
(ii) Fahren mindestens eines Anlagenteiles so, dass der Ausgangsstrom des jeweiligen Anlagenteils als Eingangsstrom dieses Anlagenteils oder eines davor liegenden Anlagenteils verwendet wird;
(iii) Außerbetriebnahme mindestens eines Anlagenteils, jedoch nicht aller Anlagenteile;
(iv) optional Öffnen des in Schritt (iii) außer Betrieb genommenen mindestens einen Anlagenteils;
(v) Durchführen einer Instandsetzungs-, Reinigungs-, Revisions- und/oder Reparaturmaßnahme in dem in Schritt (iii) außer Betrieb genommenen Anlagenteil, wobei nach Durchführung der Maßnahme die gesamte Anlage unverzüglich wieder angefahren wird und das Wiederanfahren umfasst:
(vi) optional Schließen und optional Inertisieren des mindestens einen Anlagenteils aus Schritt (v);
(vii) Inbetriebnahme des mindestens einen Anlagenteils aus Schritt (vi); und
(viii) Starten der Zufuhr des mindestens einen Substrats in den Reaktor (I);
wobei in Schritt (ii) jedes nicht außer Betrieb genommene Anlagenteil so gefahren wird, dass der Ausgangsstrom des jeweiligen Anlagenteils als Eingangsstrom dieses Anlagenteils oder eines davor liegenden Anlagenteils verwendet wird.

## Claims

1. Process for preparing a chemical product or a chemical composition, wherein the process comprises the following steps and is conducted in a plant having plant sections corresponding to these steps:
(I) reacting at least one substrate in a reactor to form at least one chemical product or a chemical composition, the at least one substrate being introduced into the reactor with a mass flow m₁;
(II) working up the reaction mixture obtained in step (I) in a workup apparatus to obtain a crude product and at least one secondary stream comprising the constituents separated from the crude product;
(III) purifying the crude product obtained in step (II) in at least one purifying apparatus to obtain a purified end product and at least one secondary stream comprising the constituents separated from the purified end product; and the optional steps (IV) to (V)
(IV) working up the at least one secondary stream obtained in step (II) in a workup unit;
(V) working up the at least one secondary stream obtained in step (III) in a workup unit,
wherein
in the event of a production shutdown lasting for 1 hour to 5 days for inspection, repair, cleaning or maintenance purposes in parts of the plant, one or more, but not all, plant sections from steps (I) to (V), if they are conducted, are put out of operation and an inspection, repair, cleaning or maintenance measure is conducted in these plant sections, and wherein
the mass flow m₁ in step (I) is reduced to zero and, in each of the plant sections that has not been shut down, the output stream of the plant section is used again as input stream for the respective plant section or an upstream plant section,
wherein, after the measure, the whole plant is started up again without delay.

2. Process according to Claim 1, comprising steps (IV) and (V).

3. Process according to at least one of the preceding claims, wherein at least two substrates are reacted in the reactor from step (I) and the second substrate is introduced into the reactor from step (I) with a mass flow m₂.

4. Process according to at least one of the preceding claims, further comprising a step (Ia), wherein at least one third substrate is reacted with the product of the reaction from step (I) in the reactor from step (I) or in a further reactor, and the at least one third substrate is introduced into the at least one reactor from step (la) with a mass flow m₃.

5. Process according to at least one of the preceding claims, wherein the chemical product is a polycarbonate or one of its precursors, an isocyanate or one of its precursors, an active pharmaceutical ingredient, an olefin, aromatic or polyolefin.

6. Method of operating a plant for preparing a chemical product or a product composition during a shutdown of the preparation process, the plant comprising the plant sections of:
(I) a reactor for execution of the conversion of at least one substrate, preferably of a plurality of (especially two) substrates,
(II) a workup apparatus for obtaining a crude product from the product mixture obtained in the reactor (I) alongside at least one secondary stream comprising the constituents separated from the crude product,
(III) a purifying apparatus for purifying the crude product obtained in the workup apparatus (II) to give a purified end product with removal of at least one secondary stream comprising the constituents separated from the purified end product, and optionally plant sections (IV) and (V):
(IV) a workup unit for workup of the at least one secondary stream obtained in the workup apparatus (II),
(V) a workup unit for workup of the at least one secondary stream obtained in the purifying apparatus (III),
the method comprising the steps of:
(i) stopping the supply of the at least one substrate, preferably all substrates, into the reactor (I),
(ii) running at least one plant section in such a way that the output stream of the respective plant section is used as input stream for that plant section or an upstream plant section;
(iii) shutting down at least one plant section, but not all plant sections;
(iv) optionally opening the at least one plant section shut down in step (iii);
(v) performing a maintenance, cleaning, inspection and/or repair measure in the plant section shut down in step (iii) wherein, after performance of the measure, the entire plant is immediately started up again, and the restart comprises:
(vi) optionally closing and optionally inertizing the at least one plant section from step (v);
(vii) starting up the at least one plant section from step (vi); and
(viii) starting the supply of the at least one substrate to the reactor (I);
wherein, in step (ii), every plant section not put out of operation is run in such a way that the output stream of the respective plant section is used again as input stream for that plant section or an upstream plant section.

## Revendications

1. Procédé de fabrication d'un produit chimique ou d'une composition chimique, le procédé comprenant les étapes suivantes et étant mis en œuvre dans une installation comprenant les parties d'installation correspondant à ces étapes :
(I) réaction d'au moins un substrat dans un réacteur avec formation d'au moins un produit chimique ou d'une composition chimique, l'au moins un substrat étant introduit dans le réacteur à un débit massique m₁ ;
(II) retraitement du mélange réactionnel obtenu dans l'étape (I) dans un appareil de retraitement avec obtention d'un produit brut et d'au moins un courant secondaire comprenant les constituants séparés du produit brut ;
(III) purification du produit brut obtenu dans l'étape (II) dans au moins un appareil de purification avec obtention d'un produit fini purifié et d'au moins un courant secondaire comprenant les constituants séparés du produit fini purifié ; et les étapes éventuelles (IV) à (V)
(IV) retraitement de l'au moins un courant secondaire obtenu dans l'étape (II) dans un équipement de retraitement ;
(V) retraitement de l'au moins un courant secondaire obtenu dans l'étape (III) dans un équipement de retraitement,
procédé dans lequel
lors d'un arrêt de production d'une durée de 1 heure à 5 heures à des fins de révision, de réparation, de nettoyage ou d'entretien dans des parties de l'installation, une ou plusieurs, mais non pas la totalité des parties de l'installation des étapes (I) à (V), dans la mesure où ces opérations sont mises en œuvre, sont mises hors service et on procède dans ces parties de l'installation à une opération de révision, de réparation, de nettoyage ou d'entretien,
le débit massique m₁ étant réduit à zéro dans l'étape (I) et, dans chacune des parties de l'installation qui ne sont pas mises hors service, on utilise de nouveau le courant de sortie de la partie de l'installation en tant que courant d'entrée de la partie de l'installation correspondante ou d'une partie de l'installation située en amont de cette dernière,
la totalité de l'installation, après ces opérations, étant redémarrée sans délai.

2. Procédé selon la revendication 1, comprenant les étapes (IV) et (V).

3. Procédé selon au moins l'une des revendications précédentes, dans lequel au moins deux substrats sont mis à réagir dans le réacteur de l'étape (I), et le second substrat est introduit dans le réacteur de l'étape (I) à un débit massique m₂.

4. Procédé selon au moins l'une des revendications précédentes, comprenant en outre une étape (Ia), au moins un troisième substrat étant mis à réagir avec le produit de la réaction de l'étape (I) dans le réacteur de l'étape (I) ou dans un autre réacteur, et l'au moins un troisième substrat étant introduit à un débit massique m₃ dans l'au moins un réacteur de l'étape (Ia).

5. Procédé selon au moins l'une des revendications précédentes, le produit chimique étant un polycarbonate ou l'un de ses précurseurs, un isocyanate ou l'un de ses précurseurs, un principe actif pharmaceutique, une oléfine, un composé aromatique ou une polyoléfine.

6. Procédé d'exploitation d'une installation destiné à la fabrication d'un produit chimique ou d'une composition de produits pendant une interruption du procédé de fabrication, l'installation comprenant les parties d'installation suivantes :
(I) un réacteur pour exécuter la réaction d'au moins un substrat, de préférence de plusieurs (en particulier deux) substrats,
(II) un appareil de retraitement pour obtenir un produit brut à partir du mélange de produits obtenu dans le réacteur (I), outre au moins un courant secondaire contenant les constituants séparés du produit brut,
(III) un appareil de purification pour purifier le produit brut obtenu dans l'appareil de retraitement (II), pour obtenir un produit fini purifié avec séparation d'au moins un courant secondaire contenant les constituants séparés du produit fini purifié, ainsi qu'éventuellement les parties d'installation (IV) à (V)
(IV) un équipement de retraitement pour retraiter l'au moins un courant secondaire obtenu dans l'appareil de retraitement (II),
(V) un équipement de retraitement pour retraiter l'au moins un courant secondaire obtenu dans l'appareil de purification (III),
le procédé comprenant les étapes suivantes :
(i) interruption de l'amenée de l'au moins un substrat, de préférence de tous les substrats, dans le réacteur (I),
(ii) exploitation d'au moins une partie de l'installation, de façon que le courant de sortie de la partie d'installation correspondante soit utilisé en tant que courant d'entrée de cette partie de l'installation et d'une partie de l'installation située en amont ;
(iii) mise hors service d'au moins une partie de l'installation, mais non pas de toutes les parties de l'installation ;
(iv) éventuellement ouverture de l'au moins une partie de l'installation mise hors service dans l'étape (iii) ;
(v) mise en œuvre d'une opération de maintenance, de nettoyage, de révision et/ou de réparation dans la partie de l'installation mise hors service dans l'étape (iii), la totalité de l'installation, après mise en œuvre de ces opérations, étant immédiatement redémarrée, le redémarrage comprenant :
(vi) éventuellement, la fermeture et éventuellement l'inertisation de l'au moins une partie d'installation de l'étape (v) ;
(vii) mise en service de l'au moins une partie de l'installation de l'étape (vi) ; et
(viii) démarrage de l'amenée de l'au moins un substrat dans le réacteur (I) ;
dans l'étape (ii) chaque partie de l'installation qui n'a pas été mise hors service étant exploitée de telle sorte que le courant de sortie de la partie de l'installation correspondante soit utilisé comme courant d'entrée de cette partie de l'installation ou d'une partie d'installation située en amont de celle-ci.
